# EUROPEAN PATENT SPECIFICATION

(11) **EP 4 138 985 B1**
(45) Date of publication and mention of the grant of the patent: **16.04.2025**
(21) Application number: 20721486.7
(22) Date of filing: 21.04.2020
(51) Int. Cl.: A61M 60/822, A61M 60/135, A61M 60/237, A61M 60/422, A61M 60/861

(54) **VENTRICULAR ASSIST DEVICE**
VENTRIKULÄRE HILFSVORRICHTUNG
DISPOSITIF D'ASSISTANCE VENTRICULAIRE

(43) Date of publication of application: 01.03.2023
(73) Proprietor: Hochschule Karlsruhe, 76133 Karlsruhe (DE); Universität Heidelberg, 69117 Heidelberg (DE)
(72) Inventor: ESTANA, Ramon, 75239 Eisingen (DE); GORENFLO, Matthias, 68799 Reilingen (DE); RUHPARWAR, Arjang, 45133 Essen (DE); KOEHLER, Felix, 76332 Bad Herrenalb (DE); POEHLER, Frank, 53604 Bad Honnef (DE); MARTENS, Eckhard, 75045 Walzbachtal (DE)
(74) Representative: Müller-Boré & Partner Patentanwälte PartG mbB
(86) International application number: PCT/EP2020/061042
(87) International publication number: WO 2021/213628

(56) References cited:
- WO-A1-2018/067410
- US-A1- 2009 118 567
- US-A1- 2016 045 652

## Description

The invention relates to a ventricular assist device. In particular the invention lies in the field of medical devices, specifically in the field of ventricular assist systems and devices.

Heart failure, chronic ischemic heart disease, and acute myocardial infarction rank amongst the top causes of death in many countries, such as for example Germany. Specifically, all three of said causes of death are linked to an impairment of the pumping function of the human heart. However, drug-based therapeutic approaches often only show a limited effect, wherein up to 25% of all patients having received stationary drug-based treatments require re-admission within 30 days. Replacing the pumping function of the heart with a donor organ is often not possible due to a lack of available donor organs. Furthermore, the use of a donor organ may in the long term lead to further complications, such as organ rejection, transplant vasculopathy, and the development of malignant neoplasms caused by the suppression of the immune response. Replacing the pumping function of the heart with an artificial pumping device, such as a ventricular assist device (VAD), is currently only temporarily possible, as further complications such as infections and embolisms may persist.

Commercially available VADs may be implemented as axial and centrifugal pumps, which may be implanted intra-corporally or extra-corporally. However, it was found that the commercially available VADs suffer from several drawbacks. For example, some commercially available VADs rely on ball bearings, which were found to cause a significant internal heat build-up and internal wear-and-tear, which contributes to a reduced lifetime of the respective VAD. Furthermore, such commercially available VADs may also produce a significant noise during their operation, which can lead to additional psychological strains for the patient.

US 2009/0118567 A1 describes methods and systems for the circulation of blood using a purge-free miniature pump. The pump is provided comprising a housing including a rotor and a stator within a drive unit. The pump establishes a primary blood flow through the space between the drive unit and the housing and a secondary blood flow between the rotor and stator. A method is provided for introducing the pump into the body and circulating blood using the pump.

US 2016/004652 A1 describes a left ventricular cardiac assist pump including an inlet opening and a discharge opening aligned in an axial direction. The pump includes a casing spaced from a stator and impeller, with at least one connecting spacer connecting the casing and the stator. The impeller is configured to direct the blood towards the casing and the discharge opening, so that blood flows through the pump principally between the casing and the rotor-stator assembly.

It is therefore an object of the present invention to provide a ventricular assist device having an improved operation and enhanced lifetime.

At least the above object is achieved by a ventricular assist device according to independent claim 1. The invention is defined by the independent claim, while preferred embodiments form the subject of the dependent claims.

One aspect of the disclosure relates to a ventricular assist device for implantation into a lumen of a blood vessel, comprising: an impeller fixed to a rotor shaft, wherein the impeller is configured to rotate around a longitudinal axis of the rotor shaft; a drive unit comprising a magnetic motor configured to cause rotation of the impeller around the longitudinal axis; a first active magnetic bearing configured to bear a first end section of the rotor shaft relative to the drive unit; a second active magnetic bearing configured to bear a second end section of the rotor shaft relative to the drive unit; and a control unit configured to control the magnetic motor, the first active magnetic bearing and the second active magnetic bearing. The blood vessel may be a vein or an artery of a user, preferentially a pulmonary artery or an aorta of the user.

The impeller is fixed to the rotor shaft, wherein the impeller is configured to rotate around a longitudinal axis of the rotor shaft. The impeller may be fixed to the rotor shaft by any means or method, such as for example by being integrally formed with the rotor shaft or by being fixed to the rotor shaft by, for example, friction fit and/or compression fit, chemical bonding, and/or using a fixing means, such as for example glues and/or corresponding engaging portions of the impeller and rotor shaft. Both impeller and rotor shaft are configured to rotate around a mutual axis, specifically the longitudinal axis of the rotor shaft. Furthermore, the ventricular assist device may be substantially cylindrically shaped and wherein the ventricular assist device may have a central axis, wherein, preferentially, the longitudinal axis of the rotor shaft may be substantially identical to the central axis of the cylindrically shaped ventricular assist device. In particular, such an impeller of a ventricular assist device of claim 1 allows for high rotational speeds, in particular in excess of 10000 rotations per minute. The impeller may further comprise at least one, preferentially six speed sensor magnets fixed to the impeller, wherein the drive unit may comprise a speed sensor, such as a speed Hall sensor, configured to detect a rotational motion of the at least one speed sensor magnets. The speed sensor may be configured to thereby determine a rotational speed of the impeller and provide said determined speed to the control unit and/or the magnetic motor.

Furthermore, the impeller may be configured to minimize turbulent flow in a fluid to be pumped by the ventricular assist device. The impeller may further be configured to reduce shear stress on the fluid to be pumped during an operation of the ventricular assist device. Furthermore, the impeller may be designed to have a high efficiency in order to minimize energy losses, which could contribute to a higher energy consumption of the ventricular assist device and/or a heating effect on the fluid to be pumped during the operation of the ventricular assist device. The impeller may be further configured to minimise damage to the fluid to be pumped, such as blood, during rotation of the impeller, wherein the impeller may in particular be configured to minimize any sharp edges of the impeller, minimize any dead water zones in a fluid field of a fluid to be pumped at and downstream of the impeller, and/or minimize zones of high shear strain in a fluid field of a fluid to be pumped at and downstream of the impeller. Furthermore, the impeller may be configured to produce a head of at least 20 mmHg, preferably at least 30 mmHg, further preferably at least 40 mmHg, and/or the impeller may be configured to produce a head of at most 150 mmHg, preferably at most 130 mmHg, further preferably at most 120 mmHg. Furthermore, the impeller may be configured to produce a volume flow of at least 1 I/min, preferably at least 2 I/min, further preferably at least 3 I/min, and/or the impeller may be configured to produce a volume flow of at most 10 I/min, preferably at most 8 I/min, further preferably at most 7 I/min. Furthermore, the impeller may comprise at least one impeller vane, wherein a number of impeller vanes of the impeller may be chosen according to specific operational requirements of the ventricular assist device. Furthermore, the impeller may comprise one or more hydraulic surfaces, wherein the hydraulic surfaces are surfaces of the impeller that may come into contact with the fluid to be pumped by the ventricular assist device during operation of the ventricular assist device. Preferentially, at least some, preferably all of the one or more hydraulic surfaces are finely polished. In other words, at least some, preferably all of the one or more hydraulic surfaces may be polished surfaces. Furthermore, the one or more hydraulic surfaces may comprise one or more surface coatings, such as one or more heparin coatings and/or one or more pyrocarbon coatings, wherein said one or more surface coatings may be configured to reduce a thrombogenicity of the one or more hydraulic surfaces. However, such surface coatings may not be restricted to hydraulic surfaces of the impeller. In particular, any surface of the ventricular assist device that may come into contact with the fluid to be pumped by the ventricular assist device during operation of the ventricular assist device may comprise one or more of the one or more surface coatings, as described above.

The drive unit comprises the magnetic motor, wherein the magnetic motor is configured to cause rotation of the impeller around the longitudinal axis. In other words, the magnetic motor is configured to generate a force on the impeller and/or the rotor shaft to cause rotation of the impeller around the longitudinal axis. In particular, the ventricular assist device may be configured such that when the ventricular assist device is placed in a fluid and the magnetic motor causes rotation of the impeller around the longitudinal axis, the rotating impeller causes a flow of the fluid relative to the ventricular assist device. Furthermore, the magnetic motor may be any kind of magnetic and/or electric motor capable of causing rotation of the impeller around the longitudinal axis.

The first active magnetic bearing is configured to bear a first end section of the rotor shaft relative to the drive unit. In particular, the adjective "active" is to be understood in this respect to indicate that the first active magnetic bearing may be actively controlled by the control unit in order to control a position of the first end section relative to the first active magnetic bearing and/or to the drive unit. The first active magnetic bearing may be further configured to control the position of the first end section relative to the first active magnetic bearing and/or to the drive unit by at least one of a permanent magnetic field and a temporary magnetic field.

The second active magnetic bearing is configured to bear a second end section of the rotor shaft relative to the drive unit. In particular, the adjective "active" is to be understood in this respect to indicate that the second active magnetic bearing may be actively controlled by the control unit in order to control a position of the second end section relative to the second active magnetic bearing and/or to the drive unit. The second active magnetic bearing may be further configured to control the position of the second end section relative to the second active magnetic bearing and/or to the drive unit by at least one of a permanent magnetic field and a temporary magnetic field.

The first end section and/or the second end section of the rotor shaft may be integrally formed with the rotor shaft. Alternatively, the first end section may comprise a first magnetisable element fixedly connected to the rotor shaft and the second end section may comprise a second magnetisable element fixedly connected to the rotor shaft, wherein the rotor shaft between the first magnetisable element and the second magnetisable element may be non-magnetisable. The first magnetisable element and/or the second magnetisable element may comprise at least one magnetisable material, e.g. magnetisable stainless steel 1.4016. The rotor shaft may further comprise a non-magnetisable section in between the first end section and the second end section, preferably in between the first magnetisable element and the second magnetisable element.

The control unit is configured to control the magnetic motor, the first active magnetic bearing and the second active magnetic bearing. The control unit may, for example, be implemented as a PID control unit configured to control the magnetic motor, the first active magnetic bearing and the second active magnetic bearing. The control unit may be implemented as a centralised unit configured to control each of the magnetic motor, the first active magnetic bearing and the second active magnetic bearing. Alternatively, the control unit may comprise one or more control sub-units, wherein each control sub-unit may be configured to control at least one of the magnetic motor, the first active magnetic bearing and the second active magnetic bearing. At least two of the one or more control sub-units may be connected to one another to transfer at least one of power and data between each other, wherein said at least two of the one or more control sub-units may be connected to one another wirelessly and/or via a physical connection, e.g. a wire connection. Alternatively or additionally, at least two of the one or more control sub-units may not be connected to one another to transfer at least one of power and data between each other, i.e. said at least two of the one or more control sub-units may be provided as separate control sub-units independent of one another. The control unit and/or the one or more control sub-units may be provided on one or more printed circuit boards (PCBs). The control unit may have one or more interfaces, such as a Bluetooth interface and/or an USB interface. Furthermore, the control unit may comprise one or more performance enhancers, such as H-bridges, for controlling magnetic bearing coils and magnetic drive coils of the ventricular assist device.

For example, the control unit may comprise at least one first control sub-unit configured to control the first active magnetic bearing, wherein the at least one first control sub-unit may be arranged proximal to, preferably on, the first active magnetic bearing. Furthermore, for example, the control unit may comprise at least one second control sub-unit configured to control the second active magnetic bearing, wherein the at least one second control sub-unit may be arranged proximal to, preferably on, the second active magnetic bearing. In particular, such an arrangement may allow for a modular design of the first and second active magnetic bearings, respectively, thereby, for example, allowing for a simplified assembly process and/or a simplified replacement of parts.

In particular, an operational state of the ventricular assist device may be a state in which the impeller is arranged relative to the drive unit such that the magnetic motor is able to cause the rotation of the impeller, in which the first end section is born by the first active magnetic bearing, and in which the second end section is born by the second active magnetic bearing.

Preferentially, the first active magnetic bearing is arranged on a first side of the drive unit, wherein the first active magnetic bearing may be fixed to the drive unit on the first side of the drive unit. Preferentially, the second active magnetic bearing is arranged on a second side of the drive unit, wherein the second active magnetic bearing may be fixed to the drive unit on the second side of the drive unit. In particular, the first side of the drive unit may be located approximately opposite to the second side of the drive unit, preferentially along a direction parallel to the central axis.

Preferentially, the control unit is configured to control the magnetic motor to adjustably generate a magnetic force on the rotor shaft to control a rotational speed of the impeller; control the first active magnetic bearing to adjustably generate a magnetic force on the first end section to control a first position of the first end section relative to the first active magnetic bearing; and control the second active magnetic bearing to adjustably generate a magnetic force on the second end section to control a second position of the second end section relative to the second active magnetic bearing.

In particular, the control unit may be configured to control the magnetic motor to adjustably generate the magnetic force on the rotor shaft to control the rotational speed of the impeller. For example, the control unit may be configured to control the magnetic motor to adjustably generate the magnetic force on the rotor shaft to increase and/or decrease the rotational speed of the impeller around the longitudinal axis. Furthermore, the control unit may be configured to control the magnetic motor to adjustably generate the magnetic force on the rotor shaft to change the direction of rotation of the impeller around the longitudinal axis.

In particular, the control unit may be configured to control the first active magnetic bearing to adjustably generate a magnetic force on the first end section, preferentially the first magnetisable element, to control a first position of the first end section relative to the first active magnetic bearing. For example, the first active magnetic bearing may comprise at least one coil, wherein the at least one coil may be adjustably supplied with a current to adjustably generate the magnetic field. Preferentially, the control unit may be configured to control the first active magnetic bearing to adjustably generate a magnetic force on the first end section, preferentially the first magnetisable element, to maintain the first position of the first end section at a first predetermined position relative to the first active magnetic bearing. The first predetermined position may in particular be a position along the central axis of the ventricular assist device.

In particular, the control unit may be configured to control the second active magnetic bearing to adjustably generate a magnetic force on the second end section, preferentially the second magnetisable element, to control a second position of the second end section relative to the second active magnetic bearing. For example, the second active magnetic bearing may comprise at least one coil, wherein the at least one coil may be adjustably supplied with a current to adjustably generate the magnetic field. Preferentially, the control unit may be configured to control the second active magnetic bearing to adjustably generate a magnetic force on the second end section, preferentially the second magnetisable element, to maintain the second position of the second end section at a second predetermined position relative to the second active magnetic bearing. The second predetermined position may in particular be a position along the central axis of the ventricular assist device.

In particular, the control unit may be configured to control the first active magnetic bearing to adjustably generate a magnetic force on the first end section and to control the second active magnetic bearing to adjustably generate a magnetic force on the second end section to maintain the longitudinal axis of the rotor shaft substantially along the central axis of the ventricular assist device in the operational state.

In particular, it may therefore be possible to significantly improve the operation of the ventricular assist device by ensuring an optimal position of the rotor shaft and impeller relative to other components of the ventricular assist device, such as the drive unit. Specifically, the flow field of the ventricular assist device during operation of said ventricular assist device can therefore be reliably maintained.

In accordance with the invention as claimed, the first active magnetic bearing may comprise a first radial magnetic bearing configured to adjust a radial position of the first end section relative to the first radial magnetic bearing, and a first radial sensor unit configured to determine the radial position of the first end section, preferentially relative to the first radial magnetic bearing. Furthermore, the first radial magnetic bearing may be configured to adjustably generate a magnetic force on the first end section to control the radial position of the first end section relative to the first radial magnetic bearing. In particular, the first radial magnetic bearing may be configured to adjustably generate a magnetic force on the first end section along a substantially radial direction relative to the longitudinal axis of the rotor shaft and/or relative to the central axis, while, preferentially, generating substantially no magnetic force on the first end section substantially parallel to the longitudinal axis of the rotor shaft and/or to the central axis. The first radial sensor unit may in particular comprise any kind of sensor capable of determining the radial position of the first end section, preferentially relative to the first radial magnetic bearing.

In accordance with the invention as claimed, the second active magnetic bearing may comprise a second radial magnetic bearing configured to adjust a radial position of the second end section relative to the second radial magnetic bearing, and a second radial sensor unit configured to determine the radial position of the second end section, preferentially relative to the second radial magnetic bearing. Furthermore, the second radial magnetic bearing may be configured to adjustably generate a magnetic force on the second end section to control the radial position of the second end section relative to the second radial magnetic bearing. In particular, the second radial magnetic bearing may be configured to adjustably generate a magnetic force on the second end section along a substantially radial direction relative to the longitudinal axis of the rotor shaft and/or relative to the central axis, while, preferentially, generating substantially no magnetic force on the second end section substantially parallel to the longitudinal axis of the rotor shaft and/or to the central axis. The second radial sensor unit may in particular comprise any kind of sensor capable of determining the radial position of the second end section, preferentially relative to the second radial magnetic bearing.

The first radial magnetic bearing may in particular comprise at least two first bearing segments, wherein said first bearing segments may be arranged circumferentially, preferentially equally spaced, around the central axis and/or the longitudinal axis of the rotor shaft in the operational state of the ventricular assist device. Each of the at least two first bearing segments may in particular be arranged substantially adjacent the rotor shaft and/or the first end section in the operational state of the ventricular assist device. Furthermore, the first radial magnetic bearing may in particular comprise at least three, preferentially at least four first bearing segments.

Furthermore, the first radial sensor unit may comprise a first radial sensor configured to measure and/or determine a capacitance between each of the first bearing segments and the first end section. In particular, the first radial sensor may be configured to measure an absolute value of the capacitance between each of the first bearing segments and the first end section and/or a change of capacitance between each of the first bearing segments and the first end section. Furthermore, the first radial sensor may be configured to determine the radial position of the first end section based on the measured capacitance between each of the first bearing segments and the first end section. The first radial sensor may comprise an operational amplifier.

In particular, the rotor shaft is not fixedly connected to the first radial magnetic bearing and can therefore move in relation to the first radial magnetic bearing during operation of the ventricular assist device. Specifically, such movement may cause the rotor shaft, in particular the first end section, to move closer to one or more first bearing segments of the at least two first bearing segments, while moving away from one or more other first bearing segments of the at least two first bearing segments. In particular, a change in the relative distances between the first end section and any of the first bearing segments causes a change in the capacitance between the first end section and the respective first bearing segment. Therefore, by measuring the capacitance, e.g. an absolute value of the capacitance and/or a change of capacitance, between each of the first bearing segments and the first end section, it may be possible to determine the distance and/or a change in distance between each of the first bearing segments and the first end section. Based on the determined distance and/or the determined change in distance between each of the first bearing segments and the first end section, it may therefore be possible to accurately derive the radial position of the first end section relative to the first radial magnetic bearing. Specifically, the first radial sensor may be configured to, in such a manner, determine the radial position of the first end section based on the measured capacitance between each of the first bearing segments and the first end section.

The second radial magnetic bearing may in particular comprise at least two second bearing segments, wherein said second bearing segments may be arranged circumferentially, preferentially equally spaced, around the central axis and/or the longitudinal axis of the rotor shaft in the operational state of the ventricular assist device. Each of the at least two second bearing segments may in particular be arranged substantially adjacent the rotor shaft and/or the second end section in the operational state of the ventricular assist device. Furthermore, the second radial magnetic bearing may in particular comprise at least three, preferentially at least four second bearing segments.

Furthermore, the second radial sensor unit may comprise a second radial sensor configured to measure and/or determine a capacitance between each of the second bearing segments and the second end section. In particular, the second radial sensor may be configured to measure an absolute value of the capacitance between each of the second bearing segments and the second end section and/or a change of capacitance between each of the second bearing segments and the second end section. Furthermore, the second radial sensor may be configured to determine the radial position of the second end section based on the measured capacitance between each of the second bearing segments and the second end section. The second radial sensor may comprise an operational amplifier.

In particular, the rotor shaft is not fixedly connected to the second radial magnetic bearing and can therefore move in relation to the second radial magnetic bearing during operation of the ventricular assist device. Specifically, such movement may cause the rotor shaft, in particular the second end section, to move closer to one or more second bearing segments of the at least two second bearing segments, while moving away from one or more other second bearing segments of the at least two second bearing segments. In particular, a change in the relative distances between the second end section and any of the second bearing segments causes a change in the capacitance between the second end section and the respective second bearing segment. Therefore, by measuring the capacitance, e.g. an absolute value of the capacitance and/or a change of capacitance, between each of the second bearing segments and the second end section, it may be possible to determine the distance and/or a change in distance between each of the second bearing segments and the second end section. Based on the determined distance and/or the determined change in distance between each of the second bearing segments and the second end section, it may therefore be possible to accurately derive the radial position of the second end section relative to the second radial magnetic bearing. Specifically, the second radial sensor may be configured to, in such a manner, determine the radial position of the second end section based on the measured capacitance between each of the second bearing segments and the second end section.

Preferentially, each of the first bearing segments comprises a magnetic yoke arranged adjacent the first end section. In particular, each of the first bearing segments may comprise at least one magnetic yoke arranged adjacent the first end section in the operational state of the ventricular assist device. The magnetic yoke may be at least partially formed from a magnetisable material, such as for example magnetisable stainless steel 1.4016. In particular, at least one, preferentially each, magnetic yoke may comprise two, preferentially equal, halves, wherein each of the halves may be formed from a magnetisable material. Furthermore, the at least one, preferentially each, magnetic yoke may further comprise an insulating plate arranged between the respective two halves, wherein the insulating plate may be configured to electrically and/or magnetically insulate the two halves from one another. In particular, a change in the relative distances between the first end section and a respective first bearing segment causes a change in the capacitance between the first end section and the respective first bearing segment, which results in a change in the capacitance between the two halves of a respective magnetic yoke. The first radial sensor may in particular be configured to measure the capacitance, e.g. an absolute value of the capacitance and/or a change of capacitance, between the two halves of the respective magnetic yoke of a first bearing segment in order to determine the distance and/or a change in distance between the respective first bearing segment and the first end section. Based on the determined distance and/or the determined change in distance between each of the first bearing segments and the first end section, it may therefore be possible to accurately derive the radial position of the first end section relative to the first radial magnetic bearing. Specifically, the first radial sensor may be configured to, in such a manner, determine the radial position of the first end section.

Preferentially, each of the first bearing segments comprises at least one first radial magnetic coil, wherein the first radial magnetic coil is wound around the magnetic yoke. In particular, the at least one first radial magnetic coil may be wound at least partially around each of the two halves of the respective magnetic yoke. Preferentially, the control unit is configured to control a current supplied to each of the first radial magnetic coils. In particular, the control unit may be configured to control a current supplied to each of the first radial magnetic coils to adjustably generate a magnetic force on the first end section to adjust the radial position of the first end section relative to the first radial magnetic bearing.

Preferentially, each of the second bearing segments comprises a magnetic yoke arranged adjacent the second end section. In particular, each of the second bearing segments may comprise at least one magnetic yoke arranged adjacent the second end section in the operational state of the ventricular assist device. The magnetic yoke may be at least partially formed from a magnetisable material, such as for example magnetisable stainless steel 1.4016. In particular, at least one, preferentially each, magnetic yoke may comprise two, preferentially equal, halves, wherein each of the halves may be formed from a magnetisable material. Furthermore, the at least one, preferentially each, magnetic yoke may further comprise an insulating plate arranged between the respective two halves, wherein the insulating plate may be configured to electrically and/or magnetically insulate the two halves from one another. In particular, a change in the relative distances between the second end section and a respective second bearing segment causes a change in the capacitance between the second end section and the respective second bearing segment, which results in a change in the capacitance between the two halves of a respective magnetic yoke. The second radial sensor may in particular be configured to measure the capacitance, e.g. an absolute value of the capacitance and/or a change of capacitance, between the two halves of the respective magnetic yoke of a second bearing segment in order to determine the distance and/or a change in distance between the respective second bearing segment and the second end section. Based on the determined distance and/or the determined change in distance between each of the second bearing segments and the second end section, it may therefore be possible to accurately derive the radial position of the second end section relative to the second radial magnetic bearing. Specifically, the second radial sensor may be configured to, in such a manner, determine the radial position of the second end section.

Preferentially, each of the second bearing segments comprises at least one second radial magnetic coil, wherein the second radial magnetic coil is wound around the magnetic yoke. In particular, the at least one second radial magnetic coil may be wound at least partially around each of the two halves of the respective magnetic yoke. Preferentially, the control unit is configured to control a current supplied to each of the second radial magnetic coils. In particular, the control unit may be configured to control a current supplied to each of the second radial magnetic coils to adjustably generate a magnetic force on the second end section to adjust the radial position of the second end section relative to the second radial magnetic bearing.

Preferentially, the at least two first bearing segments may be substantially identically constructed. Alternatively or additionally, the at least two second bearing segments may be substantially identically constructed. Alternatively or additionally, the at least two first bearing segments and the at least two second bearing segments may be substantially identically constructed. Thereby, an easy and efficient assembly and production of the ventricular assist device is made possible.

Preferentially, the at least two first bearing segments are substantially equally spaced in a circumferential direction around the longitudinal axis and/or the central axis. In particular, the at least two first bearing segments are preferentially substantially equally spaced in a circumferential direction around the longitudinal axis and/or the central axis in the operational state. Thereby, a facile and efficient magnetic bearing of the first end section may be provided. However, the disclosure is not limited to such a feature, wherein other spacings of the at least two first bearing segments are possible.

Preferentially, the at least two second bearing segments are substantially equally spaced in a circumferential direction around the longitudinal axis and/or the central axis. In particular, the at least two second bearing segments are preferentially substantially equally spaced in a circumferential direction around the longitudinal axis and/or the central axis in the operational state. Thereby, a facile and efficient magnetic bearing of the second end section may be provided. However, the disclosure is not limited to such a feature, wherein other spacings of the at least two second bearing segments are possible.

Furthermore, using the first radial sensor to determine the radial position of the first end section and/or the second radial sensor to determine the radial position of the second end section may further enhance the accuracy and efficiency of the ventricular assist device, and may further simplify the production process of the ventricular assist device. In particular, as the first radial sensor and the second radial sensor do not rely on the measurement of a magnetic field of a sensor magnet, the first radial sensor may be able to determine the radial position of the first end section and/or the second radial sensor may be able to determine the radial position of the second end section independently of a shape of the magnetic field of such a sensor magnet. Therefore, for example, effects of a non-homogeneous magnetic field of such a sensor magnet, which may cause errors in the determination of the radial positions of the first and second end sections, respectively, may be avoided and/or reduced. In particular, this may further improve the reliability and lifetime of the ventricular assist device, as the position of the rotor shaft in the ventricular assist device can be more accurately maintained.

Preferentially, the first radial sensor unit comprises a first radial Hall sensor arrangement configured to determine the radial position of the first end section. Preferentially, the first radial Hall sensor arrangement may be configured to continuously determine the radial position of the first end section or determine the radial position of the first end section at given measurement intervals. The measurement intervals may be predetermined and/or dynamically adjusted. For example, the measurement intervals may be based on a current rotational speed of the impeller, wherein a higher speed corresponds to a shorter measurement interval.

Preferentially, the second radial sensor unit comprises a second radial Hall sensor arrangement configured to determine the radial position of the second end section. Preferentially, the second radial Hall sensor arrangement may be configured to continuously determine the radial position of the second end section or determine the radial position of the second end section at given measurement intervals. The measurement intervals may be predetermined and/or dynamically adjusted. For example, the measurement intervals may be based on a current rotational speed of the impeller, wherein a higher speed corresponds to a shorter measurement interval.

The first radial Hall sensor arrangement may comprise a first permanent magnet fixed to the first end section, and at least one first radial Hall sensor arranged adjacent the first permanent magnet in a radial direction relative to the longitudinal axis, in particular relative to the longitudinal axis and/or the central axis in the operational state of the ventricular assist device. In particular, the first permanent magnet may be directly fixed to the first end section and/or rotor shaft. Alternatively the first end section may comprise a first non-magnetisable element fixedly connected to the rotor shaft and/or the first magnetisable element, wherein the first permanent magnet is fixedly connected to the first non-magnetisable element. The first radial Hall sensor arrangement may in particular comprise two or more first radial Hall sensors, wherein the two or more first radial Hall sensors are spaced, preferentially equally spaced, in a circumferential direction around the longitudinal axis, in particular around to the longitudinal axis and/or the central axis in the operational state of the ventricular assist device.

In particular, as the rotor shaft, and consequently the first permanent magnet, moves relative to the first active magnetic bearing, a distance between the first permanent magnet and one or more of the first radial Hall sensors may change, which may result in a shifted magnetic field of the first permanent magnet caused by the movement of the first permanent magnet. The one or more first radial Hall sensors may in particular be configured to measure said magnetic field and/or a change in said magnetic field. Based on said measured magnetic field and/or said measured change in the magnetic field, the one or more first radial Hall sensors and/or the first radial Hall sensor arrangement may be configured to determine a distance and/or change in distance between the first permanent magnet and each of the respective one or more first radial Hall sensors. Based on said determined distance and/or change in distance between the first permanent magnet and each of the respective one or more first radial Hall sensors, the first radial Hall sensor arrangement may in particular be configured to determine the radial position of the first end section.

Preferentially, the first permanent magnet may have a magnetic field that is substantially circular symmetric around the longitudinal axis of the rotor shaft. The first radial Hall sensor arrangement may further comprise a first calibration data unit, wherein the first calibration data unit may in particular comprise calibration data for the first radial Hall sensor arrangement. In particular, the calibration data may comprise a shape and/or strength of the magnetic field of the first permanent magnet, such as for example a vector field representation of the H-field of the first permanent magnet and/or a vector field representation of the B-field of the first permanent magnet.

In particular, the one or more first radial Hall sensors and/or the first radial Hall sensor arrangement may determine the distance and/or change in distance between the first permanent magnet and each of the respective one or more first radial Hall sensors based on the measured magnetic field and/or the measured change in the magnetic field and the calibration data. In particular, it may thereby be possible to compensate for effects of a non-circular symmetric magnetic field of the first permanent magnet on the determined position of the first end section. This may increase the reliability and the accuracy of the first radial Hall sensor arrangement, and/or allow the use of first permanent magnets, which have a magnetic field that deviates from a substantially circular symmetry around the longitudinal axis of the rotor shaft.

The second radial Hall sensor arrangement may comprise a second permanent magnet fixed to the second end section, and at least one second radial Hall sensor arranged adjacent the second permanent magnet in a radial direction relative to the longitudinal axis, in particular relative to the longitudinal axis and/or the central axis in the operational state of the ventricular assist device. In particular, the second permanent magnet may be directly fixed to the second end section and/or rotor shaft. Alternatively the second end section may comprise a second non-magnetisable element fixedly connected to the rotor shaft and/or the second magnetisable element, wherein the second permanent magnet is fixedly connected to the second non-magnetisable element. The second radial Hall sensor arrangement may in particular comprise two or more second radial Hall sensors, wherein the two or more second radial Hall sensors are spaced, preferentially equally spaced, in a circumferential direction around the longitudinal axis, in particular around to the longitudinal axis and/or the central axis in the operational state of the ventricular assist device.

In particular, as the rotor shaft, and consequently the second permanent magnet, moves relative to the second active magnetic bearing, a distance between the second permanent magnet and one or more of the second radial Hall sensors may change, which may result in a shifted magnetic field of the second permanent magnet caused by the movement of the second permanent magnet. The one or more second radial Hall sensors may in particular be configured to measure said magnetic field and/or a change in said magnetic field. Based on said measured magnetic field and/or said measured change in the magnetic field, the one or more second radial Hall sensors and/or the second radial Hall sensor arrangement may be configured to determine a distance and/or change in distance between the second permanent magnet and each of the respective one or more second radial Hall sensors. Based on said determined distance and/or change in distance between the second permanent magnet and each of the respective one or more second radial Hall sensors, the second radial Hall sensor arrangement may in particular be configured to determine the radial position of the second end section.

Preferentially, the second permanent magnet may have a magnetic field that is substantially circular symmetric around the longitudinal axis of the rotor shaft. The second radial Hall sensor arrangement may further comprise a second calibration data unit, wherein the second calibration data unit may in particular comprise calibration data for the second radial Hall sensor arrangement. In particular, the calibration data may comprise a shape and/or strength of the magnetic field of the second permanent magnet, such as for example a vector field representation of the H-field of the second permanent magnet and/or a vector field representation of the B-field of the second permanent magnet.

In particular, the one or more second radial Hall sensors and/or the second radial Hall sensor arrangement may determine the distance and/or change in distance between the second permanent magnet and each of the respective one or more second radial Hall sensors based on the measured magnetic field and/or the measured change in the magnetic field and the calibration data. In particular, it may thereby be possible to compensate for effects of a non-circular symmetric magnetic field of the second permanent magnet on the determined position of the second end section. This may increase the reliability and the accuracy of the second radial Hall sensor arrangement, and/or allow the use of second permanent magnets, which have a magnetic field that deviates from a substantially circular symmetry around the longitudinal axis of the rotor shaft.

The first radial sensor unit may be configured to provide the determined radial position of the first end section to the control unit, wherein the control unit may be configured to control the first radial magnetic bearing of the first active magnetic bearing to adjustably generate a magnetic force on the first end section on the basis of the determined radial position of the first end section. In particular, the first radial sensor unit may be configured to determine a first radial position of the first end section using the first radial sensor and to determine a second radial position of the first end section using the first radial Hall sensor arrangement. In particular, the first radial sensor unit may be configured to compare the first radial position and the second radial position of the first end section to determine the accuracy of the determined radial position of the first end section and/or to determine any error or malfunctions of the first radial sensor and the first radial Hall sensor arrangement, respectively. The first radial sensor unit may be configured to determine the radial position based on the first radial position of the first end section and/or the second radial position of the first end section. The first radial sensor unit may in particular be configured to determine the radial position of the first end section based on an arithmetic mean or a weighted average of the first radial position of the first end section and the second radial position of the first end section.

The second radial sensor unit may be configured to provide the determined radial position of the second end section to the control unit, wherein the control unit may be configured to control the second radial magnetic bearing of the second active magnetic bearing to adjustably generate a magnetic force on the second end section on the basis of the determined radial position of the second end section. In particular, the second radial sensor unit may be configured to determine a first radial position of the second end section using the second radial sensor and to determine a second radial position of the second end section using the second radial Hall sensor arrangement. In particular, the second radial sensor unit may be configured to compare the first radial position and the second radial position of the second end section to determine the accuracy of the determined radial position of the second end section and/or to determine any error or malfunctions of the second radial sensor and the second radial Hall sensor arrangement, respectively. The second radial sensor unit may be configured to determine the radial position based on the first radial position of the second end section and/or the second radial position of the second end section. The second radial sensor unit may in particular be configured to determine the radial position of the second end section based on an arithmetic mean or a weighted average of the first radial position of the second end section and the second radial position of the second end section.

Preferentially, the first radial magnetic bearing may be one of a homopolar magnetic bearing and a heteropolar magnetic bearing, and/or the second radial magnetic bearing may be one of a homopolar magnetic bearing and a heteropolar magnetic bearing.

Preferentially, the ventricular assist device may comprise an axial sensor arrangement configured to determine an axial position of the rotor shaft along the longitudinal axis and/or the central axis in the operational state. In particular, the axial sensor arrangement may comprise any type of sensor capable of detecting and/or measuring the axial position of the rotor shaft along the longitudinal axis and/or the central axis in the operational state relative to the drive unit and/or the first active magnetic bearing and/or the second active magnetic bearing.

Preferentially, the axial sensor arrangement may comprise a ring-shaped permanent magnet fixed to the rotor shaft in a circumferential direction around the rotor shaft. In particular, the ring-shaped permanent magnet may be formed from any possible magnetic material. The ring-shaped permanent magnet may be fixed to the rotor shaft between the first end section and the second end section, preferably between the first magnetisable element and the second magnetisable element.

The axial sensor arrangement may further comprise an axial Hall sensor arranged adjacent the ring-shaped permanent magnet in a direction parallel to the longitudinal axis, wherein the axial Hall sensor is configured to determine the axial position of the rotor shaft. In particular, the axial Hall sensor may be fixedly connected to the drive unit, wherein the ventricular assist device is configured such that during rotation of the impeller in the operational state the ring-shaped permanent magnet rotates adjacent to the axial Hall sensor. Therefore, an axial movement of the rotor shaft along the longitudinal axis and/or the central axis may cause a distance between the axial Hall sensor and the ring-shaped permanent magnet to change, which causes a change in the magnetic field measured by the axial Hall sensor. Based on such a measured changed magnetic field and/or change of the magnetic field, the axial Hall sensor may be configured to determine the axial position of the rotor shaft. While the axial sensor arrangement has been described using one axial Hall sensor above, the axial sensor arrangement is not restricted to this. Specifically, the axial Hall sensor arrangement may comprise one or more axial Hall sensors arranged adjacent the ring-shaped permanent magnet in a direction parallel to the longitudinal axis, wherein the one or more axial Hall sensors may further be equally spaced in a circumferential direction around the central axis. Preferentially, the axial sensor arrangement may be configured to provide the determined axial position of the rotor shaft to the control unit.

Preferentially, the first active magnetic bearing may comprise a first axial magnetic bearing configured to adjust the axial position of the rotor shaft along the longitudinal axis. In particular, the first axial magnetic bearing may be configured to adjustably generate a magnetic force on the rotor shaft to adjust the axial position of the rotor shaft along the longitudinal axis and/or the central axis. In particular, the control unit may be configured to control the first axial magnetic bearing to adjustably generate a magnetic force on the rotor shaft to adjust the axial position of the rotor shaft along the longitudinal axis and/or the central axis, preferentially based on the determined axial position of the rotor shaft.

In particular, the rotor shaft may comprise a first magnetisable disk, preferentially a first circular or ring-shaped planar magnetisable disk, fixed to the rotor shaft, preferably to the non-magnetisable section of the rotor shaft. The first magnetisable disk may be fixed to the rotor shaft such that the longitudinal axis is normal to the first magnetisable disk. The first magnetisable disk may in particular comprise at least one magnetisable material. In particular, the first axial magnetic bearing may be configured to adjustably generate a magnetic force on the first magnetisable disk to adjust the position of the rotor shaft along the longitudinal axis and/or the central axis.

The first axial magnetic bearing may further comprise a first axial magnetic coil. The first axial magnetic coil may be arranged adjacent the first magnetisable disk. The first axial magnetic coil may be wound around the longitudinal axis and/or the central axis in the operational state, wherein the first axial magnetic coil may be fixedly connected to the drive unit and/or the first active magnetic bearing. In particular, the first axial magnetic coil may be configured such that the rotor shaft is rotatable with respect to the first axial magnetic coil. The control unit may be configured to control a current to the first axial magnetic coil to adjustably generate a magnetic force on the rotor shaft to adjust the axial position of the rotor shaft along the longitudinal axis and/or the central axis, preferentially based on the determined axial position of the rotor shaft.

The first axial magnetic bearing may further comprise a first magnetic pot. The first magnetic pot may be formed from any magnetisable material. The first magnetic pot may further be configured to contain and/or surround the first axial magnetic coil, wherein the first magnetic pot may be configured to be open along a surface of the first axial magnetic coil adjacent the first magnetisable disk. In other words, the first axial magnetic coil may be arranged in the first magnetic pot, wherein the first magnetic pot may be configured to be open along a surface of the first axial magnetic coil adjacent the first magnetisable disk. In particular, it may therefore be possible to direct and/or orient a magnetic field generated by the first axial magnetic coil, thereby improving the performance of the first axial magnetic bearing.

Preferentially, the second active magnetic bearing may comprise a second axial magnetic bearing configured to adjust the axial position of the rotor shaft along the longitudinal axis. In particular, the second axial magnetic bearing may be configured to adjustably generate a magnetic force on the rotor shaft to adjust the axial position of the rotor shaft along the longitudinal axis and/or the central axis. In particular, the control unit may be configured to control the second axial magnetic bearing to adjustably generate a magnetic force on the rotor shaft to adjust the axial position of the rotor shaft along the longitudinal axis and/or the central axis, preferentially based on the determined axial position of the rotor shaft.

In particular, the rotor shaft may comprise a second magnetisable disk, preferentially a second circular or ring-shaped planar magnetisable disk, fixed to the rotor shaft, preferably to the non-magnetisable section of the rotor shaft. The second magnetisable disk may be fixed to the rotor shaft such that the longitudinal axis is normal to the second magnetisable disk. The second magnetisable disk may in particular comprise at least one magnetisable material. In particular, the second axial magnetic bearing may be configured to adjustably generate a magnetic force on the second magnetisable disk to adjust the position of the rotor shaft along the longitudinal axis and/or the central axis.

The second axial magnetic bearing may further comprise a second axial magnetic coil. The second axial magnetic coil may be arranged adjacent the second magnetisable disk. The second axial magnetic coil may be wound around the longitudinal axis and/or the central axis in the operational state, wherein the second axial magnetic coil may be fixedly connected to the drive unit and/or the second active magnetic bearing. In particular, the second axial magnetic coil may be configured such that the rotor shaft is rotatable with respect to the second axial magnetic coil. The control unit may be configured to control a current to the second axial magnetic coil to adjustably generate a magnetic force on the rotor shaft to adjust the axial position of the rotor shaft along the longitudinal axis and/or the central axis, preferentially based on the determined axial position of the rotor shaft.

The second axial magnetic bearing may further comprise a second magnetic pot. The second magnetic pot may be formed from any magnetisable material. The second magnetic pot may further be configured to contain and/or surround the second axial magnetic coil, wherein the second magnetic pot may be configured to be open along a surface of the second axial magnetic coil adjacent the second magnetisable disk. In other words, the second axial magnetic coil may be arranged in the second magnetic pot, wherein the second magnetic pot may be configured to be open along a surface of the second axial magnetic coil adjacent the second magnetisable disk. In particular, it may therefore be possible to direct and/or orient a magnetic field generated by the second axial magnetic coil, thereby improving the performance of the second axial magnetic bearing.

Furthermore, the rotor shaft may comprise a single magnetisable disk, preferentially a single circular or ring-shaped planar magnetisable disk, fixed to the rotor shaft, preferably to the non-magnetisable section of the rotor shaft. The single magnetisable disk may be fixed to the rotor shaft such that the longitudinal axis is normal to the single magnetisable disk. The single magnetisable disk may in particular comprise at least one magnetisable material. In particular, the first axial magnetic bearing may be configured to adjustably generate a magnetic force on the single magnetisable disk to adjust the position of the rotor shaft along the longitudinal axis and/or the central axis and the second axial magnetic bearing may be configured to adjustably generate a magnetic force on the single magnetisable disk to adjust the position of the rotor shaft along the longitudinal axis and/or the central axis.

Preferentially, the determined axial position is provided to the control unit. In other words, the axial sensor arrangement is configured to provide the determined axial position of the rotor shaft along the longitudinal axis and/or the central axis to the control unit. Furthermore, the control unit may be configured to control the first axial magnetic bearing of the first active magnetic bearing and/or the second axial magnetic bearing of the second active magnetic bearing to adjust the axial position of the rotor shaft on the basis of the determined axial position of the rotor shaft. For example, the control unit may control a current supplied to the first axial magnetic coil of the first axial magnetic bearing and/or to the second axial magnetic coil of the second axial magnetic bearing to adjust the axial position of the rotor shaft on the basis of the determined axial position of the rotor shaft.

Preferentially, the control unit may further comprise a transmitter configured to transmit data to a remote device, wherein the control unit is preferentially configured to detect a malfunction of the ventricular assist device and subsequently transmit an alert on the basis of the detected malfunction. For example, the control unit may be configured to detect when the ventricular assist device cannot set a rotational speed of the rotor shaft to a desired value, such as when a blockage is preventing sufficient rotation of the rotor shaft. The remote device may in particular be a handheld device, such as a smartphone. An app on said smart phone maybe configured to display the transmitted data, such as performance data of the ventricular assist device. Preferentially, the control unit may further comprise a receiver configured to receive data from a remote device. In particular, the received data may comprise for example operation instructions to the control unit, a data request to the control unit, and/or firmware updates. Preferentially, the control unit may further comprise a data storage device, wherein the data storage device may be configured to store, for example, historical operational data and/or parameters of the ventricular assist device.

Preferentially, a geometry of the ventricular assist device may be configured such that a flow field of a fluid pumped by the ventricular assist device through the ventricular assist device in the operational state does not comprise any dead water zones. In particular, a dead water zone is to be understood in this respect as a flow zone of the flow field having an average flow vector of zero. In other words, fluid located in a dead water zone of the flow field is not able to leave the dead water zone. In particular, this can cause severe problems for the operation of the ventricular assist device, as such dead water zones may significantly reduce the overall pumping performance of the ventricular assist device. Furthermore, when pumping a live fluid, such as blood, live particles of the fluid, such as blood cells, eventually consume all available nutrients and/or oxygen contained in the dead water zone, causing said live particles to eventually suffocate and/or starve. Said dead particles may then cause blood clots, which could lead to thrombosis.

Preferentially, the ventricular assist device may be configured to have a geometry such that the fluid pumped by the ventricular assist device does not flow over any sharp edges of the ventricular assist device. In other words, the ventricular assist device may be configured such that the flow field of the fluid pumped by the ventricular assist device through the ventricular assist device in the operational state has a smooth geometry. Specifically, it was found that sharp edges of the ventricular assist device may cause dead water zones, as discussed above. Furthermore, it was found that by implementing such a smooth geometry, a shear strain on the fluid to be pumped could significantly be reduced, thereby causing less potential damage to live particles in said fluid. In particular, the ventricular assist device may therefore be configured to have a geometry such that a shear strain load on the fluid to be pumped does not exceed a shear strain threshold of the fluid to be pumped during a pumping operation of the ventricular assist device and/or during rotation of the impeller. Specifically, possible geometries of the ventricular assist device may be designed and/or verified using numerical methods modelling, and/or verified using analysis of fluid pumped by the ventricular assist device, such as for example using blood damage analysis. In addition, such a geometry of the ventricular assist device may allow for a significantly improve energy conversion efficiency of up to 75%, and furthermore significantly reduces the resistance experienced by a passive flow of fluid, i.e. while the ventricular assist device is not pumping said fluid, through the ventricular assist device. An example of such a passive flow of fluid may be a flow of blood caused by the own pumping action of a heart of a user of the ventricular assist device.

Preferentially, the flow field of the fluid pumped by the ventricular assist device within the ventricular assist device in the operational state may have a variable diameter along the central axis. Specifically, the ventricular assist device may be configured such that the impeller is arranged in a portion of the flow field having the smallest diameter.

Preferentially, the ventricular assist device may further comprise a diffusor arranged adjacent the impeller. In particular, the ventricular assist device may further comprise a diffusor arranged adjacent the impeller in the operational state. The diffusor may in particular be configured to at least partially reduce and/or remove swirl produced in a fluid flow caused by the rotation of the impeller. The diffusor may further be configured to reduce and/or minimize turbulent flow in the fluid flow caused by the rotation of the impeller. In particular, such a minimized and/or reduced turbulent flow may reduce damage to a fluid to be pumped, such as blood. Furthermore, the diffusor may be configured to cause a lowest possible total pressure loss and/or a highest possible static pressure gain of a fluid pumped by the ventricular assist device. In particular, the diffusor may have a diffusor geometry, wherein the diffusor geometry may for example be determined using numerical modelling based on at least the viscosity of the fluid to be pumped and/or the outgoing flow of the impeller. Preferentially, the diffusor may comprise at least one stator vane, wherein a number of stator vanes of the diffusor may be chosen according to specific operational requirements of the ventricular assist device. Preferentially, the ventricular assist device may be configured such that the number of stator vanes of the diffusor is larger or smaller than the number of impeller vanes of the impeller.

Preferentially, the ventricular assist device may be configured to be fully implanted into the lumen of the blood vessel, such as a vein or artery, e.g. the aorta or the pulmonary artery. Fully implanted into the lumen of the blood vessel in this context is to be understood in particular as implanted into the lumen, such that after implantation the ventricular assist device is fully enclosed in said lumen. The ventricular assist device may comprise one or more attachment elements, such as one or more attachment grooves, on an outer surface of the ventricular assist device configured to fix the ventricular assist device to the blood vessel. In particular, the one or more attachment grooves may extend along a substantially circumferential direction around the central axis and/or the longitudinal axis, wherein at least one attachment groove may extend fully around the ventricular assist device and/or at least one attachment groove may extend only partially around the ventricular assist device. In particular, the one or more attachment elements may be configured to fix the ventricular assist device to a wall of the blood vessel and/or an inner surface of the wall of the blood vessel. Alternatively or additionally, the ventricular assist device may be configured to be fixable to the blood vessel, in particular to the wall of the blood vessel, by one or more fixing elements arranged outside the lumen of the blood vessel, preferentially outside the blood vessel.

For example, the one or more fixing elements may comprise one or more wires or ties configured to fix the ventricular assist device to the wall of the blood vessel and/or the inner surface of the wall of the blood vessel by at least one of friction fit and positive fit. Furthermore, the one or more wires or ties may be configured to reduce a surface pressure on the wall of the blood vessel, in order to reduce and/or avoid tissue damage, such as tissue necrosis, to said wall. In particular, said one or more wires or ties may be configured to at least partially compress the blood vessel, in particular the wall of the blood vessel between the ventricular assist device, preferentially the one or more attachment grooves of the ventricular assist device, and the respective one of the wires or ties. The number of wires or ties may be adjusted in accordance with a specific required holding force. Furthermore, the one or more wires or ties may be configured to be brought into a closed loop form by at least one of knotting, stapling, gluing, fusing, and/or the use of one or more clamps and/or brackets. The suitability of such a closed loop form has been verified by FEM (finite element method) calculations. The one or more wires or ties may be made from medical grade PTFE-tissue.

Furthermore, due to the compact design of the ventricular assist device and/or due to the ventricular assist device preferentially not comprising any physical connections, such as a wire connection, through the skin and/or the walls of the blood vessel, such as a vein or artery of a user, the ventricular assist device may be implanted in a minimally invasive procedure. In particular, such a minimally invasive procedure may not require the use of pulmonary support machines. Furthermore, the ventricular assist device may easily be further miniaturised.

Preferentially, the magnetic motor may be configured to cause rotation of the impeller, such that a pumping action of the ventricular assist device is synchronised or asynchronised with the pumping action of a user heart.

Preferentially, the magnetic motor is configured to cause rotation of the impeller in a pulsatile operation mode. During the pulsatile operation mode, the magnetic motor may be operated to cause rotation of the impeller at a first rotational speed during times when a heart of a user is working/pumping, and the magnetic motor may be operated to not cause rotation of the impeller during times when the heart is resting or to cause rotation of the impeller at a second rotational speed when the heart is resting, wherein the first rotational speed is preferentially higher than the second rotational speed. In particular, using such a pulsatile operation mode, the ventricular assist device may be able to assist the natural circulation caused by the heart. Furthermore, the user maintains having a pulse even during operation of the ventricular assist device. Furthermore, the pulsatile operation mode may prevent additional complications, such as aortic valve insufficiency, thrombus formation, and/or gastrointestinal arteriovenous malformations.

Preferentially, the magnetic motor is configured to cause rotation of the impeller in a counter-pulsatile operation mode. During the counter-pulsatile operation mode, the magnetic motor may be operated to cause rotation of the impeller at a third rotational speed during times when a heart of a user is resting, and the magnetic motor may be operated to not cause rotation of the impeller during times when the heart is working/pumping or to cause rotation of the impeller at a fourth rotational speed when the heart is working/pumping, wherein the third rotational speed is preferentially higher than the fourth rotational speed. In particular, using such a counter-pulsatile operation mode, the ventricular assist device may be able to assist the circulation caused by the heart, as well as reduce a mechanical load on the heart, specifically by providing relief for the ventricle.

Preferentially, the ventricular assist device may further comprise pulse detecting means configured to detect a pulse of a heart of a user of the ventricular assist device. The pulse detecting means may comprise remote devices configured to detect the pulse of the heart and to transmit information of the detected pulse to the ventricular assist device, and/or the pulse detecting means may comprise local devices configured to detect the pulse of the heart, such as for example pressure detecting means. In particular, the magnetic motor may be configured to cause rotation of the impeller in the pulsatile operation mode and/or the counter-pulsatile operation mode on the basis of the detected pulse of the heart of the user.

Preferentially, the magnetic motor is configured to cause rotation of the impeller in a continuous operation mode. During the continuous operation mode, the magnetic motor may be operated to cause rotation, preferentially at a constant rotational speed or at a variable rotational speed dependent for example on a current activity of the user, of the impeller during times when a heart of a user is resting and during times when the heart is working/pumping. In particular, using such a counter-pulsatile operation mode, the ventricular assist device may be able to assist the circulation caused by the heart, as well as reduce a mechanical load on the heart.

Preferentially, the magnetic motor may be configured to be switchable between the pulsatile operation mode, the counter-pulsatile operation mode, and/or the continuous operation mode.

Preferentially, the ventricular assist device comprises a power unit configured to provide power to the ventricular assist device, wherein the power unit may comprise a power reception unit configured to, preferentially wirelessly and transcutaneously, receive power. Wirelessly and transcutaneously is to be understood in this context such that the power reception unit may be configured to receive power over one or more wireless connections that do not require a physical connection, such as a wire connection, through the skin and/or the walls of the blood vessel, such as a vein or an artery of a user. However, alternatively or additionally, the power reception unit may comprise one or more physical connections, such as one or more wire connections, through the skin and/or the walls of the blood vessel, such as a vein or an artery of a user, wherein the power reception unit may be configured to receive power over the one or more physical connections. Furthermore, the power unit may comprise a power storage unit configured to store power. The power storage unit may comprise any means of storing power, such as one or more batteries and/or one or more electrostatic storage modules, such as a Goldcap capacitor.

Preferentially, the magnetic motor is a brushless DC-motor, and, optionally, wherein the brushless DC-motor has a large airgap. In particular, an imaginary plane may be perpendicular to the central axis of the ventricular assist device in the operational state, wherein all such imaginary planes intersect at least one component of the ventricular assist device. In such an imaginary plane, a total blocked area comprises all areas occupied by one or more components of the ventricular assist device in the respective imaginary plane. Furthermore, in such an imaginary plane, a total flow area comprises all areas, preferentially within the ventricular assist device, through which a fluid to be pumped can flow. Preferentially, the ventricular assist device may be configured such that the total flow area is at least 1.25 times, preferably at least 1.5 times as large as the total blocked area for any imaginary plane, as defined above. Preferentially, the ventricular assist device may be configured such that the total flow area is at least 1.25 times, preferably at least 1.5 times as large as the total blocked area for all of the imaginary planes, as defined above, that intersect at least one component of the impeller and/or the magnetic motor.

Preferentially, the brushless DC-motor has an inflow surface. Preferentially, the airgap of the brushless DC-motor has a size of at least 50%, preferably at least 60% of the inflow surface. Furthermore, the airgap of the brushless DC-motor may have a size of at least 50%, preferably at least 60% of the inflow surface in a section of the ventricular assist device, wherein said section comprises at least the magnetic motor and/or the impeller.

Preferentially, the first active magnetic bearing and the second active magnetic bearing are substantially identically constructed. In particular, at least one of the first active magnetic bearing and the second active magnetic bearing may be configured to have a mirror symmetry relative to an imaginary mirror plane perpendicular to an axis of a central bore configured to receive the first and second end sections, respectively. Thereby, a simple and efficient design of the first active magnetic bearing and the second active magnetic bearing can be provided. Alternatively, at least one of the first active magnetic bearing and the second active magnetic bearing may not have a mirror symmetry, as described above. Thereby, the design of the first active magnetic bearing and the second active magnetic bearing can be efficiently adapted to the design of the other components of the ventricular assist device.

In a preferential configuration, the ventricular assist device comprises a plurality of permanent drive magnets, preferably six permanent drive magnets, fixed to the rotor shaft in a circumferential direction around the rotor shaft. The plurality of permanent drive magnets may be fixed onto an outer circumferential surface of the rotor shaft and/or fixed into the rotor shaft such that an outer surface of the plurality of permanent drive magnets is flush with the outer surface of the rotor shaft. Preferentially, the plurality of permanent drive magnets are fixed to the rotor shaft at a location downstream of the impeller. In other words, the plurality of permanent drive magnets are fixed to the rotor shaft at a location such that during operation of the ventricular assist device, a fluid flows through the impeller prior to flowing past the plurality of permanent drive magnets. Preferentially, the plurality of permanent drive magnets are equally spaced around the rotor shaft in the circumferential direction.

Preferentially, the magnetic motor comprises a plurality of magnetic coils, preferably six magnetic coils, arranged in a circumferential direction around the rotor shaft. In particular, the plurality of magnetic coils may be configured to produce a magnetic field to interact with the plurality of permanent drive magnets to cause the rotation of the impeller relative to the longitudinal axis along the rotor shaft. Thereby the magnetic motor is able to adjustably generate a magnetic force on the rotor shaft to control a rotational speed of the impeller. However, neither the rotor shaft, nor the magnetic motor are limited to such a configuration, and other configurations to adjustably generate a magnetic force on the rotor shaft to control a rotational speed of the impeller may be implemented.

Preferentially, the control unit may comprise at least one PID controller configured to control at least one of the magnetic motor, the first active magnetic bearing, and the second active magnetic bearing. Furthermore, the control unit and/or the at least one PID controller may be implemented on one or more printed circuit boards, preferentially on one or more encapsulated printed circuit boards. Preferentially, the control unit and/or each of the at least one PID controller may be sealed from outside influences, such as sealed from the fluid to be pumped by the ventricular assist device. For example, the control unit and/or each of the at least one PID controller may each be sealed in a respective protective pod.

Preferentially, the ventricular assist device may be substantially cylindrical shaped. In particular, the ventricular assist device may have a total length along the central axis in the operational state of at most 60 mm, preferably at most 45 mm, and at least 20 mm, preferably at least 35 mm. In particular, the ventricular assist device may have a total diameter perpendicular to the central axis in the operational state of at most 40 mm, preferably at most 30 mm, and at least 10 mm, preferably at least 20 mm.

One aspect of the disclosure relates to an impeller fixed to a rotor shaft, wherein the impeller is configured to rotate around a longitudinal axis of the rotor shaft. Preferentially, the impeller may comprise any combination of the features described herein and the appended Figures.

One aspect of the disclosure relates to a drive unit comprising a magnetic motor configured to cause rotation of an impeller fixed to a rotor shaft around a longitudinal axis of the rotor shaft. Preferentially, the drive unit may comprise any combination of the features described herein and the appended Figures.

One aspect of the disclosure relates to an active magnetic bearing configured to bear an end section of a rotor shaft relative to the active magnetic bearing and/or a corresponding drive unit configured to cause rotation of the rotor shaft around a longitudinal axis thereof. Preferentially, the active magnetic bearing may comprise any combination of the features described herein and the appended Figures.

One aspect of the invention relates to a ventricular assist system, comprising a ventricular assist device. The ventricular assist device in particular may comprise: an impeller fixed to a rotor shaft, wherein the impeller is configured to rotate around a longitudinal axis of the rotor shaft; a drive unit comprising a magnetic motor configured to cause rotation of the impeller around the longitudinal axis; a first active magnetic bearing configured to bear a first end section of the rotor shaft relative to the drive unit; a second active magnetic bearing configured to bear a second end section of the rotor shaft relative to the drive unit; and a control unit configured to control the magnetic motor, the first active magnetic bearing and the second active magnetic bearing.

Preferentially, the ventricular assist device of the ventricular assist system may comprise any combination of the features of the ventricular assist devices described herein and the appended Figures, insofar as falling within the scope of the claims.

Preferentially, the ventricular assist system comprises an external power unit configured to provide power to the ventricular assist device, wherein the power unit comprises a power transmission unit configured to, preferentially wirelessly and transcutaneously, transmit power to the ventricular assist device. Wirelessly and transcutaneously is to be understood in this context such that the power transmission unit may be configured to transmit power over one or more wireless connections that do not require a wire connection through the skin and/or the walls of the blood vessel, such as a vein or artery of a user. The transmission of power may, for example, be provided via an inductive or electromagnetic field. However, alternatively or additionally, the power transmission unit may be configured to transmit power over one or more physical connections, such as one or more wire connections, through the skin and/or the walls of the blood vessel, such as a vein or an artery of a user. Preferentially, the external power unit may be configured to be wearable by a user. For example, the external power unit may be configured as a vest wearable by the user.

Preferentially, the external power unit may further comprise an external power storage unit. In particular, the external power storage unit may be configured as any type of power storage unit. Specifically, the external power storage unit may comprise at least one of a battery, such as a LiPo-accumulator, and/or a capacitor, such as a Goldcap capacitor. Preferentially, the external power storage unit is configured to be rechargeable. Preferentially, the external power unit is configured to transmit power stored in the external power storage unit via the power transmission unit to the ventricular assist device.

A further aspect of the disclosure relates to an implantation method (not claimed) of a ventricular assist device into a lumen of a blood vessel, comprising providing the ventricular assist device having any combination of features of the ventricular assist devices as described herein and/or in the appended Figures; placing the ventricular assist device fully in the lumen of the blood vessel, such as a vein or artery, preferentially the aorta or pulmonary artery, of a user; and fixing the ventricular assist device to the blood vessel and/or to a wall of the blood vessel. Preferentially, the fixing comprises compressing the blood vessel and/or the wall of the blood vessel around the ventricular assist device to prevent a movement of the ventricular assist device relative to the blood vessel. Alternatively or additionally, the fixing may further comprise providing a fixing means on the ventricular assist device, such that a movement of the ventricular assist device relative to the blood vessel is blocked. Such fixing means may for example comprise friction enhancing elements, biologically acceptable glues, and/or tissue growth elements configured to promote tissue to grow around the ventricular assist device.

Preferentially, the implantation method is not limited to the above and may comprise any combination of features described herein and the appended Figures.

The invention is further explained in reference to the appended Figures, which show illustrative, exemplary embodiments. In particular, the embodiments shown in the appended Figures are not to be interpreted as limiting the scope of the disclosure.

In particular, the Figures show:
- **Figure 1:**: a perspective, exploded view of an exemplary ventricular assist device;
- **Figure 2:**: a perspective view of a ventricular assist device 1 in the operational state;
- **Figure 3:**: a schematic cross-sectional view of an exemplary ventricular assist device 1;
- **Figure 4A:**: a cross-sectional view of a first active magnetic bearing 30A of a ventricular assist device 1;
- **Figure 4B:**: a cross-sectional view of a first active magnetic bearing 30A of Figure 4A along the line A-A;
- **Figure 4C:**: a cross-sectional view of a second active magnetic bearing 30B of a ventricular assist device 1;
- **Figure 5:**: an organ of balance 50 of a pectinid;
- **Figure 6:**: a schematic view of a first radial sensor unit of the ventricular assist device 1;
- **Figure 7:**: a schematic view of a first radial sensor unit of the ventricular assist device 1;
- **Figure 8:**: a cross-sectional view of a ventricular assist device 1;
- **Figure 9:**: a cross-sectional view of a ventricular assist device 1;
- **Figure 10:**: a FEMM (Finite Element Method Magnetics) simulation result of a magnetic field produced by an exemplary first magnetic bearing segment 33A;
- **Figure 11:**: a cross-sectional view of a fluid flow 1000 through a ventricular assist device 1;
- **Figure 12:**: the fluid field 1000 of Figure 11 as a perspective, cross-sectional 3D flow body;
- **Figure 13:**: a cross-sectional view of an exemplary ventricular assist device after implantation into a lumen of a blood vessel; and
- **Figure 14:**: a perspective view of an exemplary impeller geometry;
- **Figure 15:**: a schematic view of magnetic field lines of a permanent magnet.

In the following description of the Figures, identical components are provided with identical reference signs, unless otherwise specified for the respective figure description.

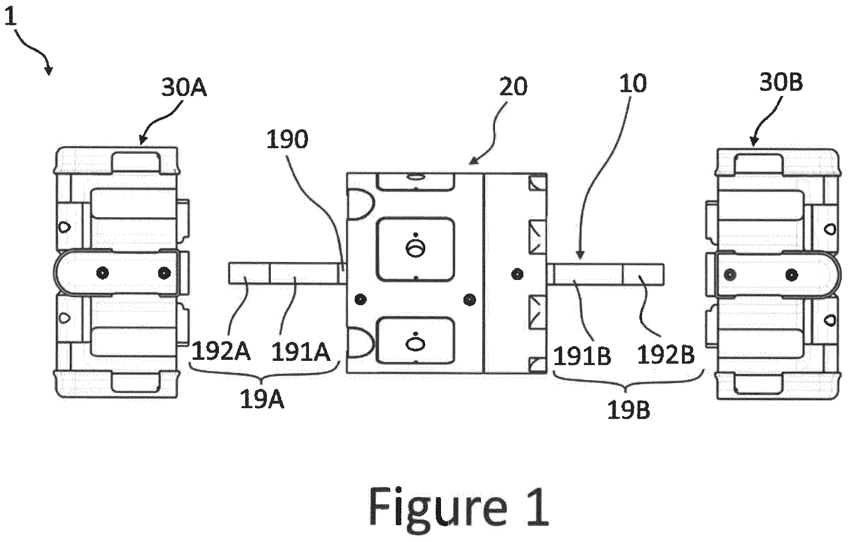

**Figure 1** shows a perspective, exploded view of an exemplary ventricular assist device **1.** The ventricular assist device **1** is shown to have a substantially cylindrical shape.

The ventricular assist device comprises in particular a rotor shaft **10.** The rotor shaft **10** is shown as arranged in the operational state of the ventricular assist device **1** in relation to the drive unit **20.** Specifically, an impeller **11** fixed to the rotor shaft **10** is arranged fully inside the drive unit **20,** and can therefore not be seen in Figure 1. The rotor shaft **10** has a longitudinal axis that is substantially identical to a central axis of the ventricular assist device **1,** wherein the rotor shaft **10** is configured to rotate around the longitudinal axis and/or the central axis. Furthermore, the rotor shaft **10** comprises a non-magnetisable section **190,** a first magnetisable element **191A,** a first non-magnetisable element **192A,** a second magnetisable element **191B,** and a second non-magnetisable element **192B.** The first magnetisable element **191A** and the first non-magnetisable element **192A** may form, in the shown embodiment, a first end section **19A** of the rotor shaft **10.** The second magnetisable element **191B** and the second non-magnetisable element **192B** may form, in the shown embodiment, a second end section **19B** of the rotor shaft **10.**

The ventricular assist device **1** further comprises the drive unit **20.** The drive unit **20** is configured to have a substantially cylindrical shape, wherein the impeller **11** of the rotor shaft **10** is arranged within the drive unit **20** in the operational state. The drive unit **20** furthermore comprises a magnetic motor (not shown in Figure 1) configured to cause rotation of the impeller **11** and the rotor shaft **10** around the longitudinal axis and the central axis during operation of the ventricular assist device **1.**

The ventricular assist device **1** further comprises a first active magnetic bearing **30A.** The first active magnetic bearing **30A** is configured to bear the first end section **19A** of the rotor shaft **10** relative to the drive unit **20** and relative to the first active magnetic bearing **30A.**

The ventricular assist device **1** further comprises a second active magnetic bearing **30B.** The second active magnetic bearing **30B** is configured to bear the second end section **19B** of the rotor shaft **10** relative to the drive unit **20** and relative to the second active magnetic bearing **30B.**

Although the first active magnetic bearing **30A** and the drive unit **20** are shown as separated for illustrative purposes in Figure 1, the first active magnetic bearing **30A** is configured to be fixedly connected to the drive unit **20** on a first side of said drive unit **20** in the operational state of the ventricular assist device **1.** Similarly, the second active magnetic bearing **30B** and the drive unit **20** are shown as separated for illustrative purposes in Figure 1, the second active magnetic bearing **30B** is configured to be fixedly connected to the drive unit **20** on a second side of said drive unit **20** in the operational state of the ventricular assist device **1.** In particular, the first side of the drive unit **20** is located opposite of the second side of the drive unit **20** along the central axis of the ventricular assist device **1.**

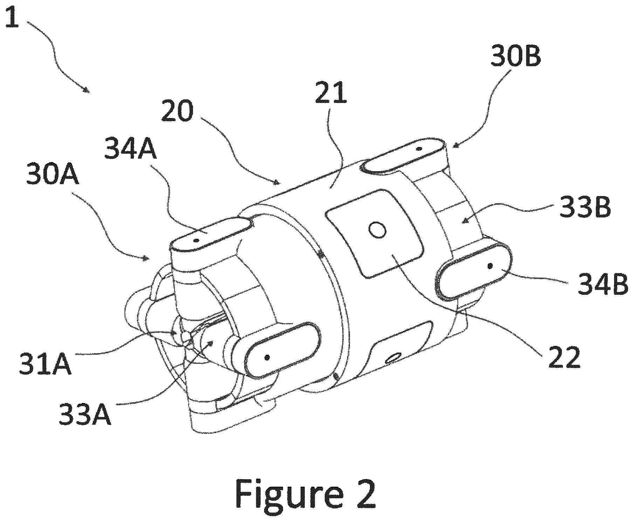

**Figure 2** shows a perspective view of a ventricular assist device **1** in the operational state.

In particular, in the shown embodiment, the first active magnetic bearing **30A** is fixedly connected to the drive unit **20.** Furthermore, the second active magnetic bearing **30B** is fixedly connected to the drive unit **20.**

The drive unit **20** may in particular comprise a, preferentially substantially cylindrical, outer body **21.** Specifically, each of the first active magnetic bearing **30A** and the second active magnetic bearing **30B** may be fixedly connected to the outer body **21.**

The drive unit may further comprise access covers **22,** wherein each access cover is removably connected to the outer body **21.** In particular, each access cover may configured to be removable to access at least the magnetic motor of the drive unit **20.**

The first active magnetic bearing **30A** may comprise in particular a first radial magnetic bearing **31A** configured to adjust a radial position of the first end section **19A** relative to the first radial magnetic bearing **31A.** The first radial magnetic bearing **31A** comprises in the shown embodiment four first bearing segments **33A,** wherein said first bearing segments **33A** are arranged circumferentially, specifically equally spaced, around the central axis and/or the longitudinal axis of the rotor shaft **10** in the operational state of the ventricular assist device **1.** Each of the four first bearing segments **33A** is in particular arranged substantially adjacent the rotor shaft **10** and/or the first end section **19A** in the operational state of the ventricular assist device **1.**

Each of the first bearing segments **33A** furthermore comprises a control unit cover **34A** located adjacent to the respective first bearing segment **33A.** Each control unit cover **34A** is in particular configured to seal a corresponding control unit and/or control sub-unit from a fluid to be pumped by the ventricular assist device **1.**

Furthermore, the four first bearing segments 33A are substantially identically constructed.

The second active magnetic bearing **30B** may comprise in particular a second radial magnetic bearing **31B** (not shown due to the perspective of Figure 2) configured to adjust a radial position of the second end section **19B** relative to the second radial magnetic bearing **31B.** The second radial magnetic bearing **31B** comprises in the shown embodiment four second bearing segments **33B,** wherein said second bearing segments **33B** are arranged circumferentially, specifically equally spaced, around the central axis and/or the longitudinal axis of the rotor shaft **10** in the operational state of the ventricular assist device **1.** Each of the four second bearing segments **33B** is in particular arranged substantially adjacent the rotor shaft **10** and/or the second end section **19B** in the operational state of the ventricular assist device **1.**

Each of the second bearing segments **33B** furthermore comprises a control unit cover **34B** located adjacent to the respective second bearing segment **33B.** Each control unit cover **34B** is in particular configured to seal a corresponding control unit and/or control sub-unit from a fluid to be pumped by the ventricular assist device **1.**

Furthermore, the four second bearing segments **33B** are substantially identically constructed.

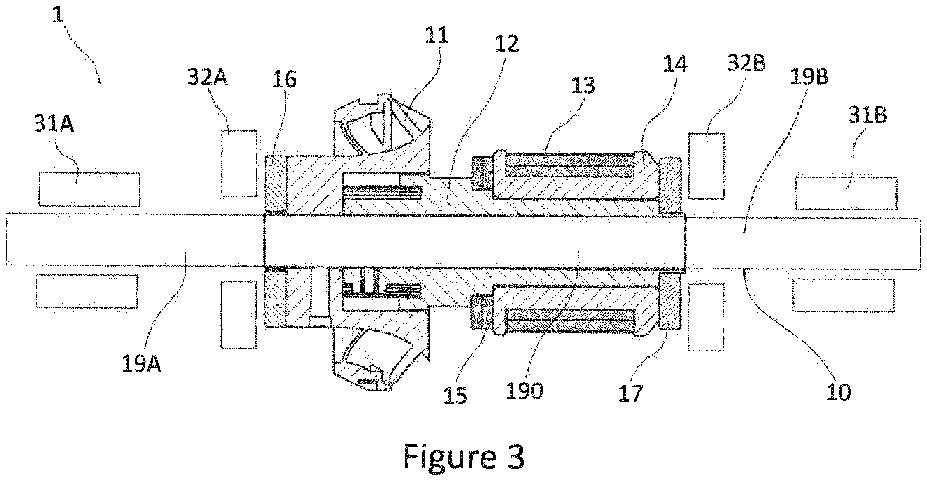

**Figure 3** shows a schematic cross-sectional view of an exemplary ventricular assist device **1.**

In particular, the first active magnetic bearing **30A** comprises at least a first radial magnetic bearing **31A** configured to bear the first end section **19A** (shown as a single end section of the rotor shaft **10** for simplicity) of the rotor shaft **10.** Furthermore, the second active magnetic bearing **30B** comprises at least a second radial magnetic bearing **31B** configured to bear the second end section **19B** (shown as a single end section of the rotor shaft **10** for simplicity) of the rotor shaft **10.**

The ventricular assist device **1** further comprises an impeller **11** fixedly connected with the rotor shaft **10,** wherein the impeller **11** is configured to be rotatable with the rotor shaft **10** around the longitudinal axis of the rotor shaft **10** and/or the central axis of the ventricular assist device **1.** The ventricular assist device **1** may further comprise a mounting body **12,** wherein the mounting body **12** is fixedly connected to the rotor shaft **10.** The mounting body **12** is in particular configured such that further elements of the ventricular assist device **1** can easily be mounted on the rotor shaft **10.** However, the mounting body **12** is not essential, and said further elements may also be directly mounted on the rotor shaft **10.**

Furthermore, the ventricular assist device **1** comprises a plurality of permanent drive magnets **13,** preferably six permanent drive magnets **13,** wherein each permanent drive magnet **13** is fixedly connected to a bracket **14.** Each bracket **14** is fixedly connected to the mounting body **12,** thereby providing a fixed connection between each of the permanent drive magnets **13** and the rotor shaft **10.** In particular, the six permanent drive magnets **13** are equally spaced in a circumferential direction around the rotor shaft **10** and/or the central axis. In particular, the plurality of permanent drive magnets **13** are fixed relative to the rotor shaft **10** at a location downstream of the impeller **11.** In other words, the plurality of permanent drive magnets **13** are fixed relative to the rotor shaft **10** at a location such that during operation of the ventricular assist device **1,** a pumped fluid flows through the impeller **11** prior to flowing past the plurality of permanent drive magnets **13.**

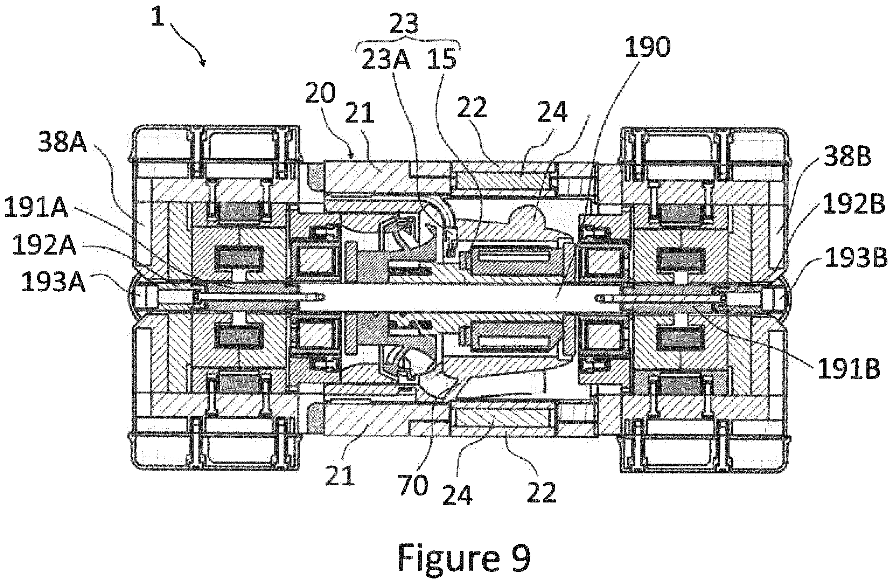

Furthermore, the ventricular assist device **1** comprises an axial sensor arrangement **23** (see for example Figure 9) configured to determine an axial position of the rotor shaft **10** along the longitudinal axis and/or the central axis in the operational state. In particular, the axial sensor arrangement **23** may comprise any type of sensor capable of detecting and/or measuring the axial position of the rotor shaft **10** along the longitudinal axis and/or the central axis in the operational state relative to the drive unit **20** and/or the first active magnetic bearing **30A** and/or the second active magnetic bearing **30B.** For simplicity, an exemplary sensor is not shown in Figure 3.

In particular, the axial sensor arrangement **23** comprises a ring-shaped permanent magnet **15** fixed relative to the rotor shaft **10** in a circumferential direction around the rotor shaft **10.** Specifically, in the shown embodiment, the ring-shaped permanent magnet **15** is fixed to the mounting body **12,** and therewith fixed to the rotor shaft **10.** Furthermore, the ring-shaped permanent magnet **15** may be formed from any possible magnetic material. The ring-shaped permanent magnet **15** may be arranged along the rotor shaft **10** at a location in between the impeller **11** and the plurality of permanent drive magnets **13.**

The axial sensor arrangement **23** may further comprise an axial Hall sensor **23A** (not shown in Figure 3) arranged adjacent the ring-shaped permanent magnet **15** in a direction parallel to the longitudinal axis, wherein the axial Hall sensor **23A** is configured to determine the axial position of the rotor shaft **10.**

The first active magnetic bearing **30A** furthermore comprises a first axial magnetic bearing **32A** configured to adjust the axial position of the rotor shaft **10** along the longitudinal axis and/or the central axis. In particular, the first axial magnetic bearing **32A** is configured to adjustably generate a magnetic force on the rotor shaft **10** to adjust the axial position of the rotor shaft **10** along the longitudinal axis and/or the central axis.

Furthermore, the rotor shaft **10** comprises a first magnetisable disk **16,** specifically a first ring-shaped planar magnetisable disk **16,** fixed to the rotor shaft **10,** preferably to the non-magnetisable section **190** of the rotor shaft 10. The first magnetisable disk **16** is fixed to the rotor shaft **10** such that the longitudinal axis is normal to the first magnetisable disk **16.** In particular, the first axial magnetic bearing **32A** may be configured to adjustably generate a magnetic force on the first magnetisable disk **16** to adjust the position of the rotor shaft **10** along the longitudinal axis and/or the central axis.

In particular, the first axial magnetic bearing **32A** may comprise a first axial magnetic coil. In particular, the first axial magnetic coil is arranged adjacent the first magnetisable disk **16.** The first axial magnetic coil is wound around the longitudinal axis and/or the central axis in the operational state. Furthermore, the first axial magnetic coil is configured such that the rotor shaft **10** is rotatable with respect to the first axial magnetic coil. Furthermore, a control unit may be configured to control a current to the first axial magnetic coil to adjustably generate a magnetic force on the rotor shaft **10** to adjust the axial position of the rotor shaft **10** along the longitudinal axis and/or the central axis, preferentially based on the axial position of the rotor shaft **10** as determined by the axial sensor arrangement **23.**

The second active magnetic bearing **30B** furthermore comprises a second axial magnetic bearing **32B** configured to adjust the axial position of the rotor shaft **10** along the longitudinal axis and/or the central axis. In particular, the second axial magnetic bearing **32B** is configured to adjustably generate a magnetic force on the rotor shaft **10** to adjust the axial position of the rotor shaft **10** along the longitudinal axis and/or the central axis.

Furthermore, the rotor shaft **10** comprises a second magnetisable disk **17,** specifically a second ring-shaped planar magnetisable disk **17,** fixed to the rotor shaft **10,** preferably to the non-magnetisable section **190** of the rotor shaft **10.** The second magnetisable disk **17** is fixed to the rotor shaft **10** such that the longitudinal axis is normal to the second magnetisable disk **17.** In particular, the second axial magnetic bearing **32B** may be configured to adjustably generate a magnetic force on the second magnetisable disk **17** to adjust the position of the rotor shaft **10** along the longitudinal axis and/or the central axis.

In particular, the second axial magnetic bearing **32B** may comprise a second axial magnetic coil. In particular, the second axial magnetic coil is arranged adjacent the second magnetisable disk **17.** The second axial magnetic coil is wound around the longitudinal axis and/or the central axis in the operational state. Furthermore, the second axial magnetic coil is configured such that the rotor shaft 10 is rotatable with respect to the second axial magnetic coil. Furthermore, a control unit may be configured to control a current to the second axial magnetic coil to adjustably generate a magnetic force on the rotor shaft **10** to adjust the axial position of the rotor shaft **10** along the longitudinal axis and/or the central axis, preferentially based on the axial position of the rotor shaft **10** as determined by the axial sensor arrangement **23.**

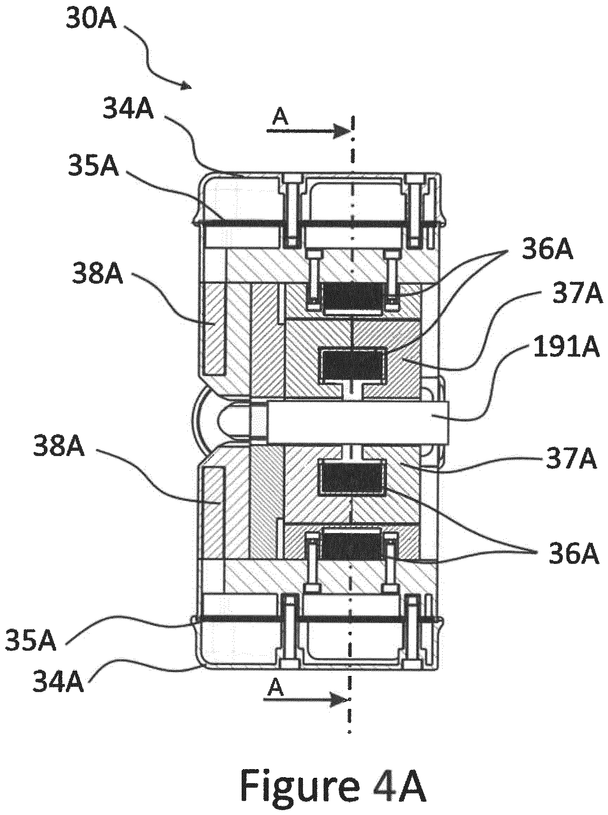

**Figure 4A** shows a cross-sectional view of a first active magnetic bearing **30A** of a ventricular assist device **1.**

In particular, the first active magnetic bearing **30A** comprises a first radial magnetic bearing **31A** configured to adjust a radial position of the first end section **19A,** specifically of the first magnetisable element **191A,** relative to the first radial magnetic bearing **31A,** and a first radial sensor unit configured to determine the radial position of the first magnetisable element **191A** relative to the first radial magnetic bearing **31A.** The first radial magnetic bearing **31A** is configured to adjustably generate a magnetic force on the first magnetisable element **191A** to control the radial position of the first magnetisable element **191A** relative to the first radial magnetic bearing **31A.** Specifically, the first radial magnetic bearing **31A** is configured to adjustably generate a magnetic force on the first magnetisable element **191A** along a substantially radial direction relative to the longitudinal axis of the rotor shaft **10** and/or relative to the central axis. Specifically, the first radial magnetic bearing **31A** is implemented as an exemplary homopolar magnetic bearing in the shown embodiment.

The first radial magnetic bearing **31A** comprises four first bearing segments **33A,** wherein said first bearing segments **33A** are arranged circumferentially and equally spaced around the central axis and/or the longitudinal axis of the rotor shaft **10** in the operational state of the ventricular assist device **1.** In the exemplary cross-section shown, two first bearing segments **33A** are illustrated. Each of the four first bearing segments **33A** is arranged substantially adjacent the rotor shaft **10,** specifically the first magnetisable element **191A,** in the operational state of the ventricular assist device **1**.

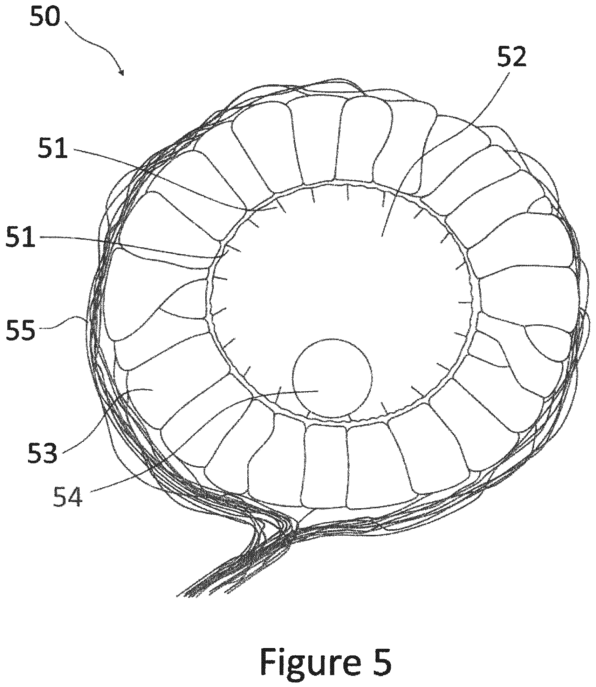

Furthermore, the first radial sensor unit comprises a first radial sensor configured to measure a capacitance between each of the first bearing segments **33A,** specifically a magnetic yoke **37A** of the respective first bearing segment **33A,** and the first magnetisable element **191A.** Said first radial sensor will be further explained with respect to Figures 5 to 7.

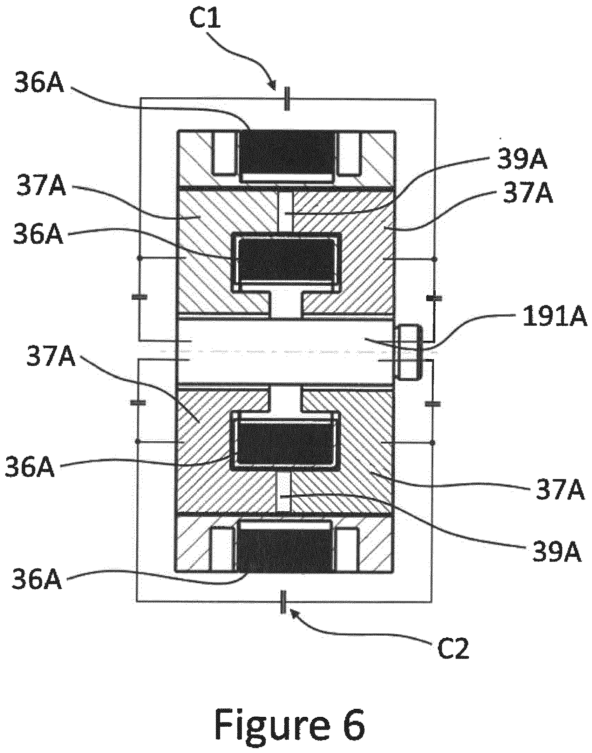

Furthermore, each of the first bearing segments **33A** comprises a magnetic yoke **37A** arranged adjacent the first magnetisable element **191A** in the operational state of the ventricular assist device **1.** The magnetic yoke **37A** may be at least partially formed from a magnetisable material, such as for example magnetisable stainless steel 1.4016. Additionally, each of the first bearing segments **33A** comprises one first radial magnetic coil **36A,** wherein said first radial magnetic coil **36A** is wound around the magnetic yoke **37A.** A close-up view of the magnetic yoke **37A** is illustrated for example in Figure 6 below.

A control unit of the ventricular assist device comprises a plurality of control sub-units **35A,** wherein each of the first bearing segments **33A** is provided with one of said control sub-units **35A.** Each control sub-unit **35A** is configured to control a current supplied to the respective first radial magnetic coil **36A** of the respective first bearing segment **33A** to adjustably generate a magnetic force on the first magnetisable element **191A** to adjust the radial position of the first magnetisable element **191A** relative to the first radial magnetic bearing **31A.** Each control sub-unit **35A** is in particular implemented on a printed circuit board, and provided with a control unit cover **34A.** Each control unit cover **34A** is in particular configured to seal the corresponding control sub-unit **35A** from a fluid to be pumped by the ventricular assist device **1.**

Furthermore, the four first bearing segments **33A** are substantially identically constructed, and substantially equally spaced in a circumferential direction around the longitudinal axis and/or the central axis.

Additionally, the first radial sensor unit comprises a first radial Hall sensor arrangement configured to determine the radial position of the first end section **19A.** In particular, the first radial Hall sensor arrangement comprises a first permanent magnet **193A** fixed to the first end section **19A,** specifically fixed to the first non-magnetisable element **192A** of the first end section **19A,** and at least one first radial Hall sensor **38A** arranged adjacent the first permanent magnet **193A** in a radial direction relative to the longitudinal axis and/or the central axis in the operational state of the ventricular assist device 1. Said first permanent magnet **193A** fixed to the first non-magnetisable element **192A** of the first end section **19A** is not shown in Figure 4A, but will be further explained with respect to Figure 9. The first radial Hall sensor arrangement comprises four first radial Hall sensors **38A,** wherein the four first radial Hall sensors **38A are** equally spaced in a circumferential direction around the longitudinal axis and/or the central axis in the operational state of the ventricular assist device **1.** Specifically, each first radial Hall sensor **38A** is arranged adjacent to one of the first bearing segments **33A.**

The first radial Hall sensor arrangement may further be shielded from the first radial magnetic bearing **31A** by one or more shielding elements arranged between the first radial Hall sensor arrangement and the first radial magnetic bearing **31A.** In particular, each first radial Hall sensor **38A** may be shielded from the respective adjacent first bearing segment **33A** by one or more shielding elements.

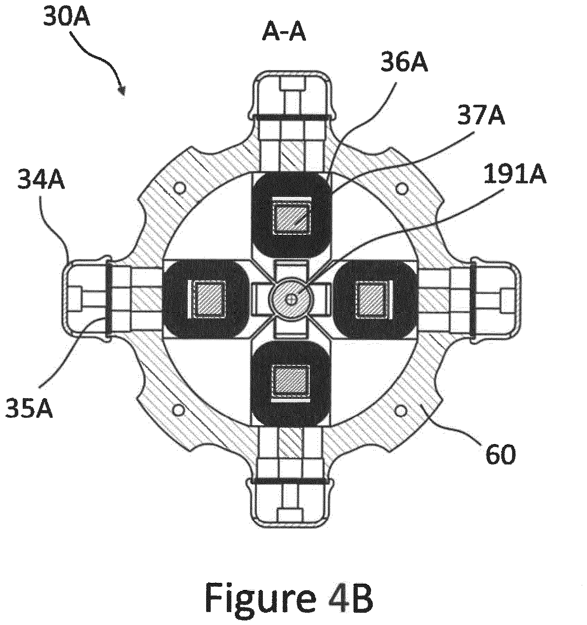

**Figure 4B** shows a cross-sectional view of a first active magnetic bearing **30A** of Figure 4A along the line A-A. For brevity, components already discussed with respect to Figure 4A will not be further discussed for Figure 4B.

The first active magnetic bearing **30A** further comprises a structural segment ring **60.** The segment ring **60** may be formed for example from a non-magnetisable material. Furthermore, each of the first bearing segments **33A** are fixedly connected to the segment ring **60.** Furthermore, each of the one or more control unit covers **34A** and/or the respective control sub-units **35A** may also be fixedly connected to the segment ring **60.** Finally, the one or more first radial Hall sensors **38A** may also be mounted on said segment ring **60.** The segment ring **60** therefore acts as a mounting platform for components of the first active magnetic bearing **30A,** and may further be fixedly connected to the drive unit **20.**

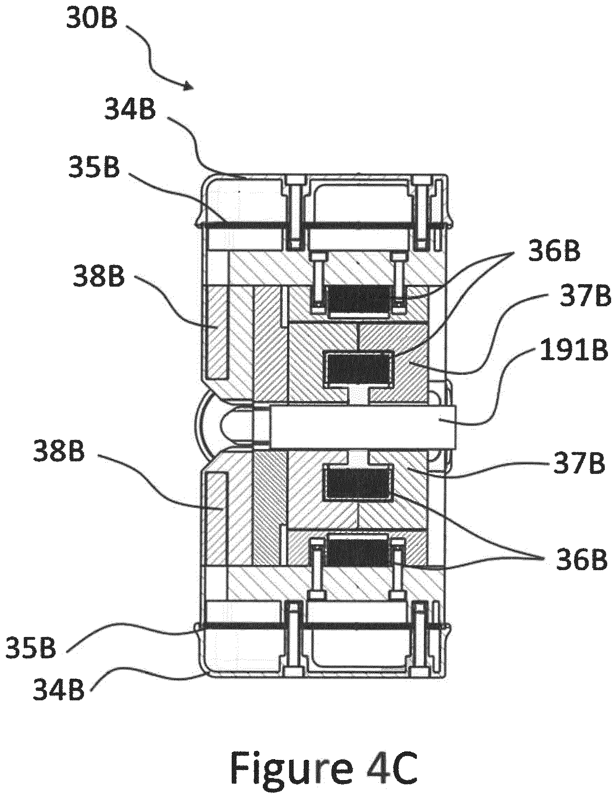

**Figure 4C** shows a cross-sectional view of a second active magnetic bearing **30B** of a ventricular assist device **1.** In particular, the second active magnetic bearing **30B** is substantially identically constructed to the first active magnetic bearing **30A.** Thereby, a facile production of the ventricular assist device can be achieved.

In particular, the second active magnetic bearing **30B** comprises a second radial magnetic bearing **31B** configured to adjust a radial position of the second end section **19B,** specifically of the second magnetisable element **191B**, relative to the second radial magnetic bearing **31B,** and a second radial sensor unit configured to determine the radial position of the second magnetisable element **191B** relative to the second radial magnetic bearing **31B.** The second radial magnetic bearing **31B** is configured to adjustably generate a magnetic force on the second magnetisable element **191B** to control the radial position of the second magnetisable element **191B** relative to the second radial magnetic bearing **31B.** Specifically, the second radial magnetic bearing **31B** is configured to adjustably generate a magnetic force on the second magnetisable element **191B** along a substantially radial direction relative to the longitudinal axis of the rotor shaft **10** and/or relative to the central axis. Specifically, the second radial magnetic bearing **31B** is implemented as an exemplary homopolar magnetic bearing in the shown embodiment.

The second radial magnetic bearing **31B** comprises four second bearing segments **33B,** wherein said second bearing segments **33B** are arranged circumferentially and equally spaced around the central axis and/or the longitudinal axis of the rotor shaft **10** in the operational state of the ventricular assist device **1.** In the exemplary cross-section shown, two second bearing segments **33B** are illustrated. Each of the four second bearing segments **33B** is arranged substantially adjacent the rotor shaft **10,** specifically the second magnetisable element **191B,** in the operational state of the ventricular assist device **1.**

Furthermore, the second radial sensor unit comprises a second radial sensor configured to measure a capacitance between each of the second bearing segments **33B,** specifically a magnetic yoke **37B** of the respective second bearing segment **33B,** and the second magnetisable element **191B**. Said second radial sensor will be further explained with respect to Figures 5 to 7.

Furthermore, each of the second bearing segments **33B** comprises a magnetic yoke **37B** arranged adjacent the second magnetisable element **191B** in the operational state of the ventricular assist device **1.** The magnetic yoke **37B** may be at least partially formed from a magnetisable material, such as for example magnetisable stainless steel 1.4016. Additionally, each of the second bearing segments **33B** comprises one second radial magnetic coil **36B,** wherein said second radial magnetic coil **36B** is wound around the magnetic yoke **37B.**

A control unit of the ventricular assist device comprises a plurality of control sub-units **35B,** wherein each of the second bearing segments **33B** is provided with one of said control sub-units **35B.** Each control sub-unit **35B** is configured to control a current supplied to the respective second radial magnetic coil **36B** of the respective second bearing segment **33B** to adjustably generate a magnetic force on the second magnetisable element **191B** to adjust the radial position of the second magnetisable element **191B** relative to the second radial magnetic bearing **31B.** Each control sub-unit **35B** is in particular implemented on a printed circuit board, and provided with a control unit cover **34B.** Each control unit cover **34B** is in particular configured to seal the corresponding control sub-unit **35B** from a fluid to be pumped by the ventricular assist device **1.**

Furthermore, the four second bearing segments **33B** are substantially identically constructed, and substantially equally spaced in a circumferential direction around the longitudinal axis and/or the central axis.

Additionally, the second radial sensor unit comprises a second radial Hall sensor arrangement configured to determine the radial position of the second end section **19B.** In particular, the second radial Hall sensor arrangement comprises a second permanent magnet **193B** fixed to the second end section **19B,** specifically fixed to the second non-magnetisable element **192B** of the second end section **19B,** and at least one second radial Hall sensor **38B** arranged adjacent the second permanent magnet **193B** in a radial direction relative to the longitudinal axis and/or the central axis in the operational state of the ventricular assist device **1.** Said second permanent magnet **193B** fixed to the second non-magnetisable element **192B** of the second end section **19B** is not shown in Figure 4C, but will be further explained with respect to Figure 9. The second radial Hall sensor arrangement comprises four second radial Hall sensors **38B,** wherein the four second radial Hall sensors **38B** are equally spaced in a circumferential direction around the longitudinal axis and/or the central axis in the operational state of the ventricular assist device **1.** Specifically, each second radial Hall sensor **38B** is arranged adjacent to one of the second bearing segments **33B.**

The second radial Hall sensor arrangement may further be shielded from the second radial magnetic bearing **31B** by one or more shielding elements arranged between the second radial Hall sensor arrangement and the second radial magnetic bearing **31B.** In particular, each second radial Hall sensor **38B** may be shielded from the respective adjacent second bearing segment **33B** by one or more shielding elements.

The second active magnetic bearing **30B** may further also comprise a structural segment ring **60.** The segment ring **60** may be formed for example from a non-magnetisable material. Furthermore, each of the second bearing segments **33B** are fixedly connected to the segment ring **60.** Furthermore, each of the one or more control unit covers **34B** and/or the respective control sub-units **35B** may also be fixedly connected to the segment ring **60.** Finally, the one or more second radial Hall sensors **38B** may also be mounted on said segment ring **60.** The segment ring **60** therefore acts as a mounting platform for components of the second active magnetic bearing **30B,** and may further be fixedly connected to the drive unit **20.**

**Figure 5** shows an organ of balance **50** of a pectinid. Specifically, said organ of balance **50** served as the inspiration for the first radial sensor and the second radial sensor.

In particular, the organ of balance **50** of the pectinid comprises a fluid-filled central cavity **52,** wherein a movable otolith **54** is arranged. The inner surface of the central cavity **52** is further lined with hair-like receptors **51,** wherein each hair-like receptor **51** is connected to at least one receptor cell **53** of the cavity wall. As the otolith **54** changes position within the central cavity **52,** the otolith **54** comes into contact with one or more of said hair-like receptors **51,** which consequently generate a neural signal. The neural signals are transmitted over the receptor cells **53** and thereto connected neural pathways **55.** Said neural signals are subsequently evaluated by a neural network.

Analogous to this, the first radial sensor may, for example, rely on a measured change in capacitance between the first magnetisable element **191A** and a respective first bearing segment **33A,** caused by a movement of the rotor shaft **10** with respect to the first radial sensor. Based on the measured change in capacitance, the first radial sensor consequently determines the position of the first end section **19A** and/or the first magnetisable element **191A** relative to the first radial magnetic bearing **31A.**

**Figure 6** shows a schematic view of a first radial sensor unit of the ventricular assist device **1,** wherein the first radial magnetic bearing **31A** is implemented as an exemplary homopolar magnetic bearing in the shown embodiment.

In particular, the first radial sensor unit may comprise a first radial sensor configured to measure a capacitance between each of the first bearing segments **33A** and the first end section **19A,** specifically the first magnetisable element **191A.** Figure 6 shows two of the four first bearing segments **33A** of the first radial magnetic bearing **31A.** Each first bearing segment **33A** comprises a first magnetic coil **36A** and a magnetic yoke **37A,** wherein the first magnetic coil **36A** is wound around the magnetic yoke **37A.** Furthermore, the magnetic yoke **37A** is separated into two equal halves, wherein said equal halves are separated from one another by an insulating plate **39A.**

In particular, the first radial sensor may be configured to measure an absolute value of the capacitance between each of the first bearing segments **33A** and the first magnetisable element **191A** and/or a change of capacitance between each of the first bearing segments **33A** and the first magnetisable element **191A.** Furthermore, the first radial sensor is configured to determine the radial position of the first magnetisable element **191A** based on the measured capacitance between each of the first bearing segments **33A** and the first magnetisable element **191A.**

In particular, the rotor shaft **10** is not fixedly connected to the first radial magnetic bearing **31A** and can therefore move in relation to the first radial magnetic bearing **31A** during operation of the ventricular assist device **1.** Specifically, such movement may cause the rotor shaft **10,** in particular the first magnetisable element **191A,** to move closer to one or more first bearing segments **33A** of the four first bearing segments **33A,** while moving away from one or more other first bearing segments **33A** of the four first bearing segments **33A.** In particular, a change in the relative distances between the first magnetisable element **191A** and any of the first bearing segments **33A** causes a change in the capacitance between the first magnetisable element **191A** and the respective first bearing segment **33A.** Specifically, the capacitance may be measured between the two halves of the magnetic yoke **37A,** i.e. at measuring points **C1** and **C2.** Therefore, by measuring the capacitance at measuring points **C1** and **C2** (and the other two corresponding measuring points of the two other first bearing segments not shown) it is possible to determine the distance and/or a change in distance between each of the first bearing segments **33A** and the first magnetisable element **191A.** Based on the determined distance and/or the determined change in distance between each of the first bearing segments **33A** and the first magnetisable element **191A,** it is therefore possible to accurately derive the radial position of the first magnetisable element **191A** relative to the first radial magnetic bearing **31A.**

Therefore, the first radial sensor provides four measuring points for the capacitance, while the second radial sensor may provide an additional four measuring points. The equations for determining the radial position of the rotor shaft are therefore an over-constrained system, which allows for an increased accuracy in the determination of said radial position.

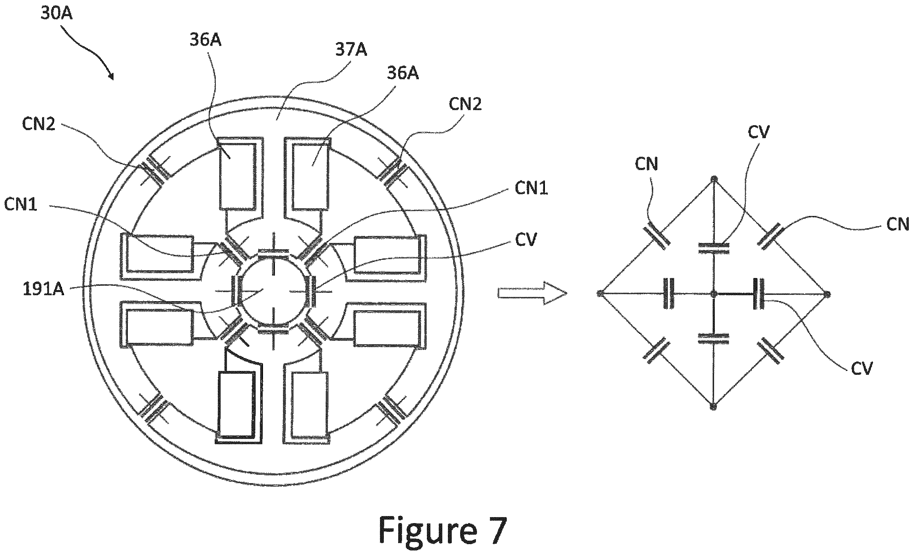

**Figure 7** shows a schematic view of a first radial sensor unit of the ventricular assist device **1,** wherein the first radial magnetic bearing **31A** is implemented as an exemplary heteropolar magnetic bearing in the shown embodiment, and a corresponding schematic capacitor map.

In particular, the first radial sensor unit may comprise a first radial sensor configured to measure a capacitance between each of the first bearing segments **33A** and the first end section **19A,** specifically the first magnetisable element **191A.** Figure 7 shows all four of the first bearing segments **33A** of the first radial magnetic bearing **31A.** Each first bearing segment **33A** comprises a first magnetic coil **36A** and a magnetic yoke **37A,** wherein the first magnetic coil **36A** is wound around the magnetic yoke **37A.** Furthermore, each magnetic yoke **37A** is separated from and insulated against its respectively neighbouring magnetic yokes **37A** by four insulating plates **39A** (not shown in Figure 7 for simplicity).

In particular, the first radial sensor may be configured to measure an absolute value of the capacitance between each of the first bearing segments **33A** and the first magnetisable element **191A** and/or a change of capacitance between each of the first bearing segments **33A** and the first magnetisable element **191A.** Furthermore, the first radial sensor is configured to determine the radial position of the first magnetisable element **191A** based on the measured capacitance between each of the first bearing segments **33A** and the first magnetisable element **191A.** Specifically, the four magnetic yokes **37A** are fixedly arranged in relation to one another, and are furthermore electrically insulated against one another. Therefore, in the shown embodiment, each two neighbouring magnetic yokes **37A** form a first non-variable capacitor **CN1** and a second non-variable capacitor **CN2,** forming a combined non-variable capacitor **CN.** In particular, the capacitance of the first non-variable capacitor **CN1** and the second non-variable capacitor **CN2** does not change with the movement of the rotor shaft **10** and/or the first magnetisable element **191A.** In particular, insulating plates **39A** may be arranged within each of the first non-variable capacitors **CN1** and/or within each of the second non-variable capacitors **CN2.**

In particular, the rotor shaft **10** is not fixedly connected to the first radial magnetic bearing **31A** and can therefore move in relation to the first radial magnetic bearing **31A** during operation of the ventricular assist device **1.** Specifically, such movement may cause the rotor shaft **10,** in particular the first magnetisable element **191A,** to move closer to one or more first bearing segments **33A** of the four first bearing segments **33A,** while moving away from one or more other first bearing segments **33A** of the four first bearing segments **33A.** In particular, a change in the relative distances between the first magnetisable element **191A** and any of the first bearing segments **33A** causes a change in the capacitance between the first magnetisable element **191A** and the respective first bearing segment **33A.** In other words, each of the magnetic yokes **37A** and the first magnetisable element **191A** form a variable capacitor **CV.** Therefore, by measuring the capacitance across each of the variable capacitor **CV,** it is possible to determine the distance and/or a change in distance between each of the first bearing segments **33A** and the first magnetisable element **191A.** Based on the determined distance and/or the determined change in distance between each of the first bearing segments **33A** and the first magnetisable element **191A,** it is therefore possible to accurately derive the radial position of the first magnetisable element **191A** relative to the first radial magnetic bearing **31A.**

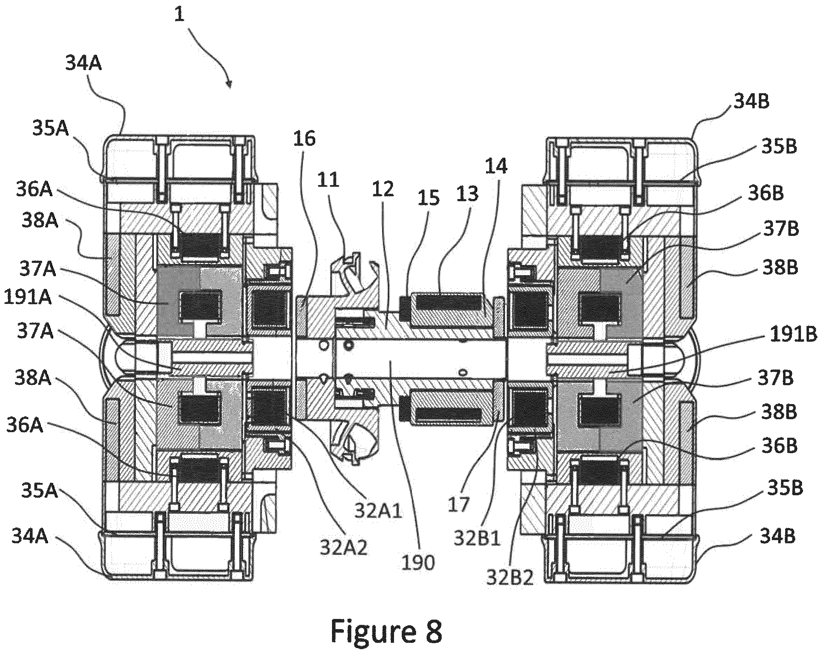

**Figure 8** shows a cross-sectional view of a ventricular assist device 1, wherein the drive unit 20 has been removed from said ventricular assist device 1. With respect to components already discussed above, reference is made to the corresponding description sections.

The first axial magnetic bearing **32A** comprises a first axial magnetic coil **32A1.** The first axial magnetic coil **32A1** is arranged adjacent the first magnetisable disk **16.** The first axial magnetic coil **32A1** is wound around the longitudinal axis and/or the central axis in the operational state, wherein the first axial magnetic coil **32A1** is fixedly connected to the first active magnetic bearing **30A.** In particular, the first axial magnetic coil **32A1** is configured such that the rotor shaft **10,** and in particular the non-magnetisable section **190** is rotatable with respect to the first axial magnetic coil **32A1.** The control unit, preferentially at least one of the control sub-units **35A,** may be configured to control a current to the first axial magnetic coil **32A1** to adjustably generate a magnetic force on the rotor shaft **10** via the first magnetisable disk **16** to adjust the axial position of the rotor shaft **10** along the longitudinal axis and/or the central axis, preferentially based on the determined axial position of the rotor shaft **10.**

The first axial magnetic bearing **32A** further comprises a first magnetic pot **32A2.** The first magnetic pot **32A2** may be formed from any magnetisable material. The first magnetic pot **32A2** is configured to contain and/or surround the first axial magnetic coil **32A1,** wherein the first magnetic pot **32A2** is configured to be open along a surface of the first axial magnetic coil **32A1** adjacent the first magnetisable disk **16.** In other words, the first axial magnetic coil **32A1** is arranged in the first magnetic pot **32A2,** wherein the first magnetic pot **32A2** is configured to be open along a surface of the first axial magnetic coil **32A1** adjacent the first magnetisable disk **16.** In particular, it is therefore possible to direct and/or orient a magnetic field generated by the first axial magnetic coil **32A1,** thereby improving the performance of the first axial magnetic bearing **32A.**

The second axial magnetic bearing **32B** comprises a second axial magnetic coil **32B1.** The second axial magnetic coil **32B1** is arranged adjacent the second magnetisable disk **17.** The second axial magnetic coil **32B1** is wound around the longitudinal axis and/or the central axis in the operational state, wherein the second axial magnetic coil **32B1** is fixedly connected to the second active magnetic bearing **30B.** In particular, the second axial magnetic coil **32B1** is configured such that the rotor shaft **10,** and in particular the non-magnetisable section **190** is rotatable with respect to the second axial magnetic coil **32B1.** The control unit, preferentially at least one of the control sub-units **35B,** may be configured to control a current to the second axial magnetic coil **32B1** to adjustably generate a magnetic force on the rotor shaft **10** via the second magnetisable disk **17** to adjust the axial position of the rotor shaft **10** along the longitudinal axis and/or the central axis, preferentially based on the determined axial position of the rotor shaft **10.**

The second axial magnetic bearing **32B** further comprises a second magnetic pot **32B2.** The second magnetic pot **32B2** may be formed from any magnetisable material. The second magnetic pot **32B2** is configured to contain and/or surround the second axial magnetic coil **32B1,** wherein the second magnetic pot **32B2** is configured to be open along a surface of the second axial magnetic coil **32B1** adjacent the second magnetisable disk **17.** In other words, the second axial magnetic coil **32B1** is arranged in the second magnetic pot **32B2,** wherein the second magnetic pot **32B2** is configured to be open along a surface of the second axial magnetic coil **32B1** adjacent the second magnetisable disk **17.** In particular, it is therefore possible to direct and/or orient a magnetic field generated by the second axial magnetic coil **32B1,** thereby improving the performance of the second axial magnetic bearing **32B.**

**Figure 9** shows a cross-sectional view of a ventricular assist device **1,** as shown in Figure 8, wherein said ventricular assist device further comprises the drive unit **20.** With respect to components already discussed above, reference is made to the corresponding description sections. In particular, for reasons of clarity, reference signs are provided for some features not included in Figure 8.

The ventricular assist device **1** comprises an axial sensor arrangement **23** (not marked in Figure 9) configured to determine an axial position of the rotor shaft **10** along the longitudinal axis and/or the central axis in the operational state.

In particular, the axial sensor arrangement **23** comprises a ring-shaped permanent magnet **15** fixed relative to the rotor shaft **10** in a circumferential direction around the rotor shaft **10.** Specifically, in the shown embodiment, the ring-shaped permanent magnet **15** is fixed to the mounting body **12,** and therewith fixed to the rotor shaft **10.** Furthermore, the ring-shaped permanent magnet **15** may be formed from any possible magnetic material. The ring-shaped permanent magnet **15** may be arranged along the rotor shaft **10** at a location in between the impeller **11** and the plurality of permanent drive magnets **13.**

The axial sensor arrangement **23** further comprises an axial Hall sensor **23A** arranged adjacent the ring-shaped permanent magnet **15** in a direction parallel to the longitudinal axis, wherein the axial Hall sensor **23A** is configured to determine the axial position of the rotor shaft **10.**

In particular, the axial Hall sensor **23A** is fixedly connected to the drive unit **20,** wherein the ventricular assist device **1** is configured such that during rotation of the impeller **11** in the operational state the ring-shaped permanent magnet **15** rotates adjacent to the axial Hall sensor **23A.** Therefore, an axial movement of the rotor shaft **10** along the longitudinal axis and/or the central axis may cause a distance between the axial Hall sensor **23A** and the ring-shaped permanent magnet **15** to change, which causes a change in the magnetic field measured by the axial Hall sensor **23A.** Based on such a measured changed magnetic field and/or change of the magnetic field, the axial Hall sensor **23A** is configured to determine the axial position of the rotor shaft **10.**

The drive unit **20** comprises the magnetic motor, wherein the magnetic motor is configured to cause rotation of the impeller **11** around the longitudinal axis. In particular, the magnetic motor of the drive unit **20** comprises a plurality of magnetic coils **24,** preferably six magnetic coils **24,** arranged in a circumferential direction around the rotor shaft **10** in the operational state. In particular, the plurality of magnetic coils **24** are configured to produce a magnetic field to interact with the plurality of permanent drive magnets **13** to cause the rotation of the impeller **11** relative to the longitudinal axis along the rotor shaft **10.** Thereby the magnetic motor is able to adjustably generate a magnetic force on the rotor shaft **10** to control a rotational speed of the impeller **11.** Each of the plurality of magnetic coils may be provided in a recess in the outer body **21** of the drive unit **20,** wherein each of the plurality of magnetic coils **24** is covered in the operational state by a respective access cover **22** of the drive unit. Each access cover **22** may be removably attached to the outer body **21** of the drive unit **20** to substantially seal off the respective recess.

The ventricular assist device **1** further comprises a diffusor **70** arranged adjacent the impeller **11.** The diffusor **70** is in particular configured to at least partially reduce and/or remove any swirl produced in a fluid flow caused by the rotation of the impeller **11.** Furthermore, the diffusor as described herein may be further configured aid in the orientation and straightening of the flow generated by the impeller.

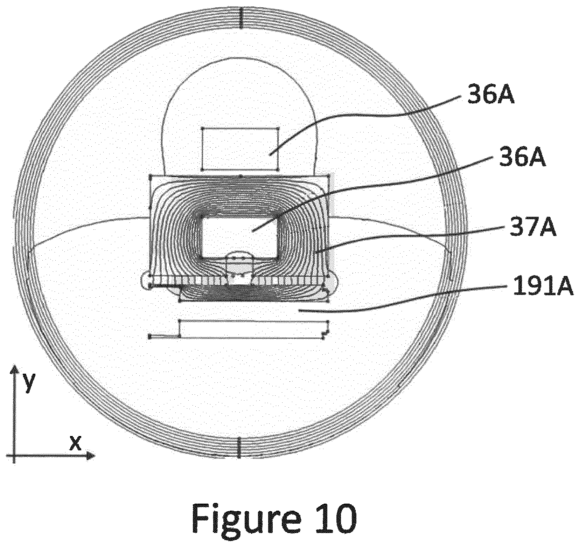

**Figure 10** shows a FEMM (Finite Element Method Magnetics) simulation result of a magnetic field produced by an exemplary first magnetic bearing segment **33A,** having a first axial magnetic coil **36A** (both the upper cross-section and the lower cross-section of the first axial magnetic coil have been labelled with 36A) wound around a first magnetic yoke **37A,** on a first magnetisable element **191A.** For the purposes of the simulation, the first magnetic yoke **37A** and the first magnetisable element **191A** are simulated as being formed from magnetisable stainless steel type 1.4016. Furthermore, for the purposes of the simulation, the first axial magnetic coil **36A** comprises 450 windings of copper wire having a wire thickness of 0.5 mm, wherein the first axial magnetic coil **36A** has a depth of 15 mm, and wherein a current of 1 A is passed through the first axial magnetic coil **36A.** Specifically, the simulation determined a net force on the first magnetisable element **191A** of 22.1574 Newton along the y-direction of the coordinate system shown in Figure 10. Furthermore, the simulation determined a net force on the first magnetic element **191A** of -0.005048 Newton along the x-direction of the coordinate system shown in Figure 10. Therefore, the exemplary first magnetic bearing segment **33A** is shown to be able to generate a sufficient magnetic force on the rotor shaft **10** and/or the first magnetisable element **191A** to adjust a radial position of the first magnetisable element **191A** and/or the rotor shaft **10** relative to the first active magnetic bearing **30A.** In particular, the exemplary first magnetic bearing segment **33A** can generate said sufficient magnetic force in the y-direction, while substantially not generating a perpendicular force on the first magnetisable element **191A.**

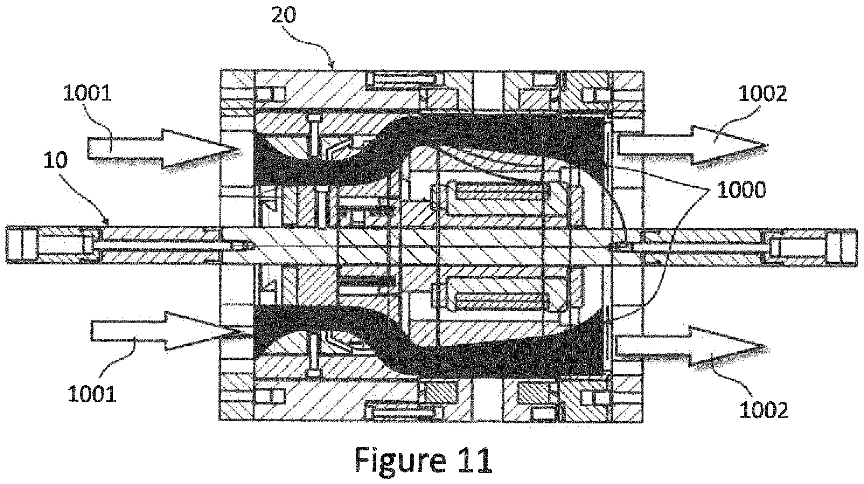

**Figure 11** shows a cross-sectional view of a fluid flow **1000** through a ventricular assist device **1,** wherein only the rotor shaft **10** and the drive unit **20** are shown for simplicity. In particular, the fluid flow **1000** is herein represented by the black flow field, while inflow **1001** is schematically represented by two arrows on the left-hand side of Figure 11 and outflow **1002** is schematically represented by two arrows on the right-hand side of Figure 11.

Specifically, the geometry of the ventricular assist device **1** is configured such that the flow field **1000** of a fluid pumped by the ventricular assist device **1** through the ventricular assist device **1** in the operational state does not comprise any dead water zones. In particular, said dead water zones are specifically prevented in the ventricular assist device **1** due to the ventricular assist device **1** being configured to have a geometry such that the fluid pumped by the ventricular assist device **1** does not flow over any sharp edges of the ventricular assist device **1.** Thereby, a shear strain on the fluid to be pumped is further significantly reduced, thus causing less damage to live particles in said fluid.

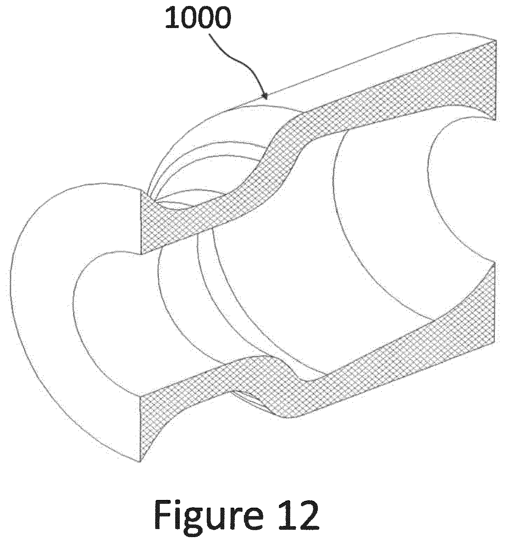

**Figure 12** shows the fluid field **1000** of Figure 11 as a perspective, cross-sectional 3D flow body. Specifically, the consistently smooth shape of the flow field **1000** can easily be observed.

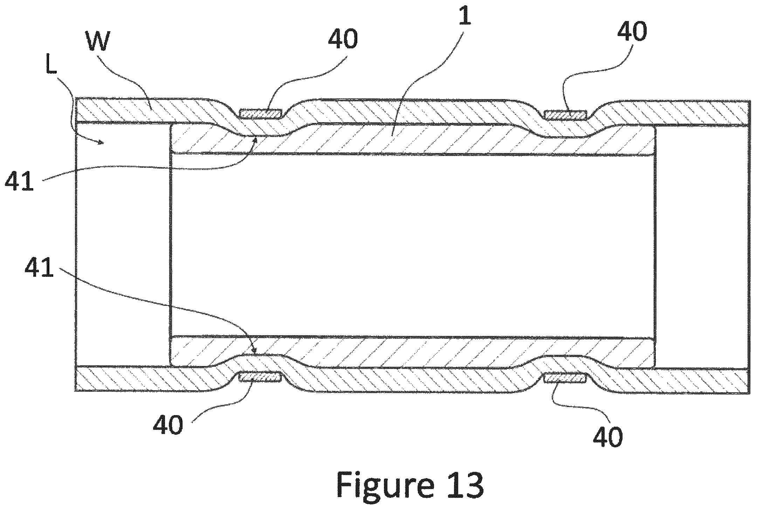

**Figure 13** shows a cross-sectional view of an exemplary ventricular assist device **1** after implantation into a lumen **L** of a blood vessel, such as a vein or artery. In particular, the blood vessel may for example be an aorta or pulmonary artery of a user.

In particular, the ventricular assist device **1** has been fully implanted into the lumen **L** of the blood vessel. Specifically, the ventricular assist device **1** has been implanted into the lumen **L** of the blood vessel, such that the ventricular assist device **1** is fully enclosed in said lumen **L.** Furthermore, the ventricular assist device **1** is configured to wirelessly and transcutaneously receive power and/or transmission signals from outside the lumen **L** of the blood vessel. In particular, the ventricular assist device **1** does not comprise a physical connection, such as a wire connection, through the walls **W** of the blood vessel.

The ventricular assist device **1** furthermore comprises two exemplary attachment grooves **41** on an outer surface of the ventricular assist device **1.** Each of the two attachment grooves **41** extends fully around the ventricular assist device **1** in a circumferential direction relative to the central axis. However, the invention is not to be restricted to such a number of possible attachment grooves **41.** In particular, the ventricular assist device **1** may comprise one, two, or more attachment grooves **41,** wherein the number of attachment grooves **41** may be adapted to specific requirements for the ventricular assist device **1,** such as a shape and/or wall thickness of the lumen in which the ventricular assist device **1** is to be implanted.

Figure 13 further shows two fixing elements **40,** wherein said fixing elements **40** may each be a wire or tie. In particular, the two fixing elements **40** are configured to at least partially compress the wall **W** of the blood vessel between the ventricular assist device **1** and the respective fixing element **40.** Specifically, each of the two fixing elements **40** is arranged adjacent to one of the two attachment grooves **41,** such that the two fixing elements **40** are configured to at least partially compress the wall **W** of the blood vessel in the respective attachment groove **41** of the ventricular assist device **1,** thereby preventing the ventricular assist device **1** from moving along the lumen **L** of the blood vessel.

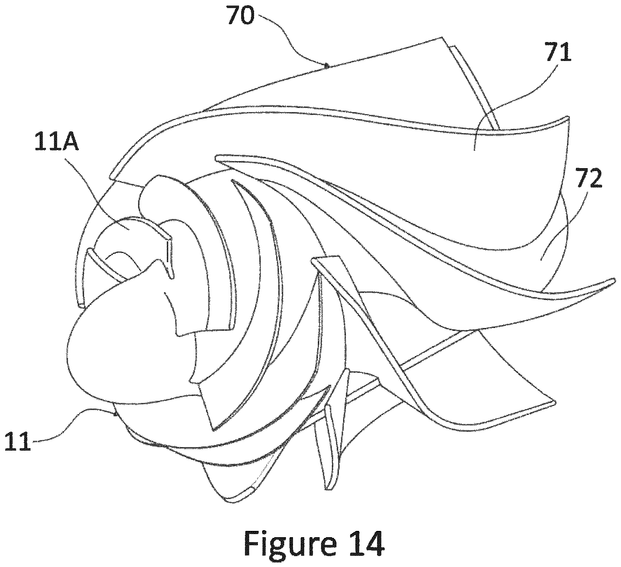

**Figure 14** shows a perspective view of an exemplary impeller **11** of the ventricular assist device **1** having an exemplary impeller geometry. Specifically, for illustrative purposes, the impeller **11** of Figure 14 is shown separate from the rotor shaft **10.**

In particular, the impeller **11** may be formed on or connected to the rotor shaft **10.** The impeller **11** may comprise a plurality of first impeller vanes **11A** at a first end of the impeller **11.** The plurality of first impeller vanes **11A** may in particular be configured such that during rotation of the impeller **11** (in the shown example in the clockwise direction when viewed from the first end of the impeller **11**) a fluid, in which the impeller **11** is placed, is pumped from the first end of the impeller **11** towards a second end of the impeller **11.**

Furthermore, Figure 14 shows a perspective view of an exemplary diffusor **70** having an exemplary diffusor geometry, wherein the diffusor **70** may be arranged adjacent the impeller **11.** In particular, the diffusor **70** may be formed around the rotor shaft **10,** wherein the diffusor **70** may be configured to be static with respect to the rotor shaft **10** and the impeller **11.** In particular, the diffusor **10** may be fixedly connected to the drive unit **20** and/or one or more components of the drive unit **20.** In other words, the diffusor **70** may be configured to be static relative to the impeller **11** even during rotation of the impeller **11.** Therefore, in particular, the exemplary diffusor **70** may be physically separate from the exemplary impeller **11.** Furthermore, the diffusor **70** may be formed around the rotor shaft **10,** wherein the diffusor **70** may comprise a base body **72,** wherein the base body **72** may be configured to have a substantially hollow cylindrical shape. In particular, in an operational state of the ventricular assist device **1** the rotor shaft **10** may be at least partially arranged within the base body **72.** In particular, the base body **72** may further be configured such that a fluid pumped by the impeller **11** is prevented from coming into direct, physical contact with the rotor shaft **10** at least while flowing through the diffusor **70.**

The exemplary diffusor **70** may comprise one or more stator vanes **71.** The exemplary geometry of the diffusor **70** and/or a geometry of the stator vanes **71** may in particular be configured to at least partially reduce and/or minimize swirl and/or turbulent flow produced in a fluid flow caused by the rotation of the impeller **11.** Furthermore, the exemplary geometry of the diffusor **70** and/or a geometry of the stator vanes **71** may be configured to cause a lowest possible total pressure loss and/or a highest possible static pressure gain of a fluid pumped by the ventricular assist device **1.** The one or more stator vanes **71** may be fixedly connected to the base body **72.**

Furthermore, in the shown embodiment, a number of stator vanes **71** is different from a number of impeller vanes **11A.** Specifically, in the shown example, the number of stator vanes **71** is seven and the number of impeller vanes **11A** is six. However, the invention is not to be restricted to such a ratio. In particular, the number of stator vanes **71** may be larger or smaller than the number of impeller vanes **11A.** Furthermore, the number of stator vanes **71** may be identical to the number of impeller vanes **11A.**

The shown impeller geometry and the shown diffusor geometry is to be understood as exemplary. Specifically, a plurality of different impeller and diffusor geometries may be implemented.

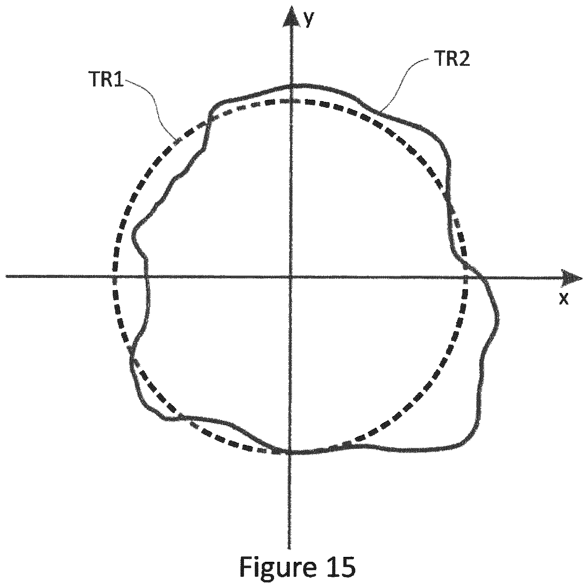

**Figure 15** shows a coordinate system highlighting a difference between an ideal permanent magnet and a commonly produced permanent magnet.

In particular, for the shown example, both the ideal permanent magnet and the commonly produced permanent magnet are cylindrically shaped. However, for clarity of the figure, neither one of the magnets is shown in Figure 15. Furthermore, both the ideal permanent magnet and the commonly produced permanent magnet are centred on the origin of the shown coordinate system, such that an axis of both the ideal permanent magnet and the commonly produced permanent magnet is perpendicular to the coordinate axes shown.

In particular, the ideal permanent magnet produces a magnetic field that is circular symmetric around the axis of the shown ideal permanent magnet, as represented by the dashed circular trace **T1.** However, commonly produced permanent magnets often contain one or more defects, which cause a distortion of a magnetic field generated by the commonly produced permanent magnet, as represented by the solid trace **T2,** when compared to the magnetic field generated by the ideal permanent magnet.

When such a commonly produced permanent magnet is used as a sensor magnet, for example in combination with a Hall sensor, such distortions may result in errors in the measurement signal of the respective sensor. However, this may be addressed by using calibration data, as discussed above, wherein the calibration data may comprise a shape and/or strength of the magnetic field of the commonly produced permanent magnet. The respective sensor may in particular be configured to use the calibration data to account for and thereby compensate possible distortions of the magnetic field.

The embodiments described herein and/or shown in the appended Figures are not to be interpreted as limiting the scope of the invention. Therefore, for example, a ventricular assist device may comprise any combination of features described herein and/or shown in the appended Figures, insofar as falling within the scope of the claims.

### List of Reference Numerals

- **1**: Ventricular assist device
- **10**: Rotor shaft
- **11**: Impeller
- **11A**: Impeller vanes
- **12**: Mounting body
- **13**: Permanent drive magnet
- **14**: Bracket
- **15**: Ring-shaped permanent magnet
- **16**: First magnetisable disk
- **17**: Second magnetisable disk
- **19A**: First end section
- **19B**: Second end section
- **190**: Non-magnetisable section
- **191A**: First magnetisable element
- **191B**: Second magnetisable element
- **192A**: First non-magnetisable element
- **192B**: Second non-magnetisable element
- **193A**: First permanent magnet
- **193B**: Second permanent magnet
- **20**: Drive unit
- **21**: Outer body
- **22**: Access cover
- **23**: Axial sensor arrangement
- **23A**: Axial Hall sensor
- **24**: Plurality of magnetic coils
- **30A**: First active magnetic bearing
- **30B**: Second active magnetic bearing
- **31A**: First radial magnetic bearing
- **31B**: Second radial magnetic bearing
- **32A**: First axial magnetic bearing
- **32B**: Second axial magnetic bearing
- **32A1**: First axial magnetic coil
- **32B1**: Second axial magnetic coil
- **32A2**: First magnetic pot
- **32B2**: Second magnetic pot
- **33A**: First bearing segment
- **33B**: Second bearing segment
- **34A, 34B**: Control unit cover
- **35A**: Control sub-unit
- **36A**: First radial magnetic coil
- **36B**: Second radial magnetic coil
- **37A, 37B**: Magnetic yoke
- **38A**: First radial Hall sensor
- **38B**: Second radial Hall sensor
- **40**: Fixing elements
- **41**: Attachment grooves
- **50**: Organ of balance
- **51**: Hair-like receptor
- **52**: Central cavity
- **53**: Receptor cell
- **54**: Otolith
- **55**: Neural pathway
- **60**: Structural segment ring
- **70**: Diffusor
- **71**: Stator vanes
- **72**: Hollow cylindrical base body
- **1000**: Fluid flow
- **1001**: Inflow
- **1002**: Outflow
- **C1, C2**: Measuring point
- **CN, CN1, CN2**: Non-variable capacitor
- **CV**: Variable capacitor
- **L**: Lumen
- **T1, T2**: Trace
- **W**: Wall

## Claims

1. Ventricular assist device (1) for implantation into a lumen (L) of a blood vessel, comprising:
an impeller (11) fixed to a rotor shaft (10), wherein the impeller (11) is configured to rotate around a longitudinal axis of the rotor shaft (10);
a drive unit (20) comprising a magnetic motor configured to cause rotation of the impeller (11) around the longitudinal axis;
a first active magnetic bearing (30A) configured to bear a first end section (19A) of the rotor shaft (10) relative to the drive unit (20);
a second active magnetic bearing (30B) configured to bear a second end section (19B) of the rotor shaft (10) relative to the drive unit (20); and
a control unit configured to control the magnetic motor, the first active magnetic bearing (30A) and the second active magnetic bearing (30B),
wherein the first active magnetic bearing (30A) comprises:
a first radial magnetic bearing (31A) configured to adjust a radial position of the first end section (19A) relative to the first radial magnetic bearing (31A),
a first radial sensor unit configured to determine the radial position of the first end section (19A); and/or
wherein the second active magnetic bearing (30B) comprises:
a second radial magnetic bearing (31B) configured to adjust a radial position of the second end section (19B) relative to the second radial magnetic bearing (31B),
a second radial sensor unit configured to determine the radial position of the second end section (19B).

2. Ventricular assist device (1) according to claim 1, wherein the control unit is configured to:
control the magnetic motor to adjustably generate a magnetic force on the rotor shaft (10) to control a rotational speed of the impeller (11); and/or
control the first active magnetic bearing (30A) to adjustably generate a magnetic force on the first end section (19A) to control a first position of the first end section (19A) relative to the first active magnetic bearing (30A); and/or
control the second active magnetic bearing (30B) to adjustably generate a magnetic force on the second end section (19B) to control a second position of the second end section (19B) relative to the second active magnetic bearing (30B).

3. Ventricular assist device (1) according to one of the preceding claims,
wherein the first radial magnetic bearing (31A) comprises at least two first bearing segments (33A),
wherein the first radial sensor unit comprises a first radial sensor configured to measure a capacitance between each of the first bearing segments (33A) and the first end section (19A),
wherein the first radial sensor is configured to determine the radial position of the first end section (19A) based on the measured capacitance between each of the first bearing segments (33A) and the first end section (19A); and/or
wherein the second radial magnetic bearing (31B) comprises at least two second bearing segments (33B),
wherein the second radial sensor unit comprises a second radial sensor configured to measure a capacitance between each of the second bearing segments (33B) and the second end section (19B),
wherein the second radial sensor is configured to determine the radial position of the second end section (19B) based on the measured capacitance between each of the second bearing segments (33B) and the second end section (19B).

4. Ventricular assist device (1) according to claim 3, wherein each of the first and second bearing segments (33A, 33B) comprises:
a magnetic yoke (37A, 37B) arranged adjacent the first and second end section (19A, 19B), respectively; and
a radial magnetic coil (36A, 36B), wherein the radial magnetic coil (36A, 36B) is wound around the magnetic yoke (37A, 37B).

5. Ventricular assist device (1) according to claim 3 or 4, wherein the at least two first bearing segments (33A) are substantially identically constructed; and/or
wherein the at least two second bearing segments (33B) are substantially identically constructed; and/or
wherein the at least two first bearing segments (33A) are substantially equally spaced in a circumferential direction around the longitudinal axis; and/or
wherein the at least two second bearing segments (33B) are substantially equally spaced in a circumferential direction around the longitudinal axis.

6. Ventricular assist device (1) according to one of the preceding claims, wherein:
the first radial sensor unit comprises a first radial Hall sensor arrangement configured to determine the radial position of the first end section (19A); and/or
the second radial sensor unit comprises a second radial Hall sensor arrangement configured to determine the radial position of the second end section (19B).

7. Ventricular assist device (1) according to claim 6, wherein the first radial Hall sensor arrangement comprises a first permanent magnet (193A) fixed to the first end section (19A), and at least one first radial Hall sensor (38A) arranged adjacent the first permanent magnet (193A) in a radial direction relative to the longitudinal axis, and/or
wherein the second radial Hall sensor arrangement comprises a second permanent magnet (193B) fixed to the second end section (19B), and at least one second radial Hall sensor (38B) arranged adjacent the second permanent magnet (193B) in the radial direction relative to the longitudinal axis.

8. Ventricular assist device (1) according to one of the preceding claims, wherein the first radial sensor unit is configured to provide the determined radial position of the first end section (19A) to the control unit,
wherein the control unit is configured to control the first radial magnetic bearing (31A) of the first active magnetic bearing (30A) to adjustably generate a magnetic force on the first end section (19A) on the basis of the determined radial position of the first end section (19A); and/or
wherein the second radial sensor unit is configured to provide the determined radial position of the second end section (19B) to the control unit, and
wherein the control unit is configured to control the second radial magnetic bearing (31B) of the second active magnetic bearing (30B) to adjustably generate a magnetic force on the second end section (19B) on the basis of the determined radial position of the second end section (19B).

9. Ventricular assist device (1) according to one of the preceding claims, wherein the first radial magnetic bearing (31A) is one of a homopolar magnetic bearing and a heteropolar magnetic bearing, and/or
wherein the second radial magnetic bearing (31B) is one of a homopolar magnetic bearing and a heteropolar magnetic bearing.

10. Ventricular assist device (1) according to one of the preceding claims, wherein the ventricular assist device (1) comprises an axial sensor arrangement (23) configured to determine an axial position of the rotor shaft (10) along the longitudinal axis, and, optionally,
wherein the axial sensor arrangement (23) comprises:
a ring-shaped permanent magnet (15) fixed to the rotor shaft (10) in a circumferential direction around the rotor shaft (10),
an axial Hall sensor (23A) arranged adjacent the ring-shaped permanent magnet (15) in a direction parallel to the longitudinal axis, wherein the axial Hall sensor (23A) is configured to determine the axial position of the rotor shaft (10).

11. Ventricular assist device (1) according to claim 10, wherein the first active magnetic bearing (30A) comprises a first axial magnetic bearing (32A) configured to adjust the axial position of the rotor shaft (10) along the longitudinal axis, and/or
wherein the second active magnetic bearing (30B) comprises a second axial magnetic bearing (32B) configured to adjust the axial position of the rotor shaft (10) along the longitudinal axis;
and, optionally:
wherein the determined axial position is provided to the control unit, wherein the control unit is configured to control the first axial magnetic bearing (32A) of the first active magnetic bearing (30A) and/or the second axial magnetic bearing (32B) of the second active magnetic bearing (30B) to adjust the axial position of the rotor shaft (10) on the basis of the determined axial position of the rotor shaft (10).

12. Ventricular assist device (1) according to one of the preceding claims, wherein the control unit comprises:
- a transmitter configured to transmit data to a remote device, wherein the control unit is preferentially configured to detect a malfunction of the ventricular assist device (1) and subsequently transmit an alert on the basis of the detected malfunction; and/or
- a receiver configured to receive data from a remote device; and/or
- a data storage device, wherein the data storage device is configured to store historical operational data of the ventricular assist device (1).

13. Ventricular assist device (1) according to one of the preceding claims, wherein a geometry of the ventricular assist device (1) is configured such that a flow field of a fluid pumped by the ventricular assist device (1) does not comprise any dead water zones, wherein the geometry of the ventricular assist device (1) is preferentially configured such that the fluid pumped by the ventricular assist device (1) does not flow over any sharp edges of the ventricular assist device (1); and/or
wherein the magnetic motor is configured to cause rotation of the impeller (11) in at least one of a pulsatile operation mode, a counter-pulsatile operation mode, and a continuous operation mode; and/or
wherein the ventricular assist device (1) comprises a power unit configured to provide power to the ventricular assist device (1), wherein the power unit comprises at least one of a power reception unit configured to, preferentially wirelessly and transcutaneously, receive power, and a power storage unit configured to store power.

14. Ventricular assist device (1) according to one of the preceding claims, wherein the magnetic motor is a brushless DC-motor, wherein the brushless DC-motor preferably has a large airgap, and/or
wherein the first active magnetic bearing (30A) and the second active magnetic bearing (30B) are substantially identically constructed; and/or
wherein the blood vessel is one of a vein and an artery of a user, preferentially a pulmonary artery or an aorta of the user.

15. Ventricular assist device (1) according to one of the preceding claims, wherein the ventricular assist device (1) comprises one or more attachment elements on an outer surface of the ventricular assist device (1) configured to fix the ventricular assist device (1) to the blood vessel; and/or
wherein the ventricular assist device (1) is configured to be fixable to the blood vessel by one or more fixing elements (40) arranged outside the lumen (L) of the blood vessel; and/or
wherein the ventricular assist device (1) is configured to be fully implanted into the lumen (L) of the blood vessel.

## Patentansprüche

1. Eine ventrikuläre Unterstützungsvorrichtung (1) zur Implantation in ein Lumen (L) eines Blutgefäßes, Folgendes umfassend:
ein Laufrad (11), das an einer Rotorwelle (10) befestigt ist, wobei das Laufrad (11) konfiguriert ist, um sich um eine Längsachse der Rotorwelle (10) zu drehen;
eine Antriebseinheit (20), umfassend einen Magnetmotor, der konfiguriert ist, um eine Drehung des Laufrads (11) um die Längsachse zu bewirken;
ein erstes aktives Magnetlager (30A), das konfiguriert ist, um einen ersten Endabschnitt (19A) der Rotorwelle (10) relativ zur Antriebseinheit (20) zu lagern;
ein zweites aktives Magnetlager (30B), das konfiguriert ist, um einen zweiten Endabschnitt (19B) der Rotorwelle (10) relativ zur Antriebseinheit (20) zu lagern; und
eine Steuereinheit, die konfiguriert ist, um den Magnetmotor, das erste aktive Magnetlager (30A) und das zweite aktive Magnetlager (30B) zu steuern,
wobei das erste aktive Magnetlager (30A) Folgendes umfasst:
ein erstes radiales Magnetlager (31A), das konfiguriert ist, um eine radiale Position des ersten Endabschnitts (19A) relativ zum ersten radialen Magnetlager (31A) einzustellen,
eine erste radiale Sensoreinheit, die konfiguriert ist, um die radiale Position des ersten Endabschnitts (19A) zu bestimmen;
und/oder
wobei das zweite aktive Magnetlager (30B) Folgendes umfasst:
ein zweites radiales Magnetlager (31B), das konfiguriert ist, um eine radiale Position des zweiten Endabschnitts (19B) relativ zum zweiten radialen Magnetlager (31B) einzustellen,
eine zweite radiale Sensoreinheit, die konfiguriert ist, um die radiale Position des zweiten Endabschnitts (19B) zu bestimmen.

2. Die ventrikuläre Unterstützungsvorrichtung (1) nach Anspruch 1, wobei die Steuereinheit konfiguriert ist, um Folgendes zu bewerkstelligen:
Steuern des Magnetmotors, um einstellbar eine Magnetkraft auf die Rotorwelle (10) zu erzeugen, um eine Drehzahl des Laufrads (11) zu steuern; und/oder
Steuern des ersten aktiven Magnetlagers (30A), um einstellbar eine Magnetkraft auf den ersten Endabschnitt (19A) zu erzeugen, um eine erste Position des ersten Endabschnitts (19A) relativ zum ersten aktiven Magnetlager (30A) zu steuern; und/oder
Steuern des zweiten aktiven Magnetlagers (30B), um einstellbar eine Magnetkraft auf den zweiten Endabschnitt (19B) zu erzeugen, um eine zweite Position des zweiten Endabschnitts (19B) relativ zum zweiten aktiven Magnetlager (30B) zu steuern.

3. Die ventrikuläre Unterstützungsvorrichtung (1) nach einem der vorstehenden Ansprüche,
wobei das erste radiale Magnetlager (31A) mindestens zwei erste Lagersegmente (33A) umfasst,
wobei die erste radiale Sensoreinheit einen ersten radialen Sensor umfasst, der konfiguriert ist, um eine Kapazität zwischen jedem von den ersten Lagersegmenten (33A) und dem ersten Endabschnitt (19A) zu messen,
wobei der erste radiale Sensor konfiguriert ist, um die radiale Position des ersten Endabschnitts (19A) basierend auf der gemessenen Kapazität zwischen jedem von den ersten Lagersegmenten (33A) und dem ersten Endabschnitt (19A) zu bestimmen; und/oder
wobei das zweite radiale Magnetlager (31B) mindestens zwei zweite Lagersegmente (33B) umfasst,
wobei die zweite radiale Sensoreinheit einen zweiten radialen Sensor umfasst, der konfiguriert ist, um eine Kapazität zwischen jedem von den zweiten Lagersegmenten (33B) und dem zweiten Endabschnitt (19B) zu messen,
wobei der zweite radiale Sensor konfiguriert ist, um die radiale Position des zweiten Endabschnitts (19B) basierend auf der gemessenen Kapazität zwischen jedem von den zweiten Lagersegmenten (33B) und dem zweiten Endabschnitt (19B) zu bestimmen.

4. Die ventrikuläre Unterstützungsvorrichtung (1) nach Anspruch 3, wobei jedes von den ersten und zweiten Lagersegmenten (33A, 33B) Folgendes umfasst:
ein Magnetjoch (37A, 37B), das angrenzend an den ersten bzw. zweiten Endabschnitt (19A, 19B) angeordnet ist; und
eine radiale Magnetspule (36A, 36B), wobei die radiale Magnetspule (36A, 36B) um das Magnetjoch (37A, 37B) gewickelt ist.

5. Die ventrikuläre Unterstützungsvorrichtung (1) nach Anspruch 3 oder 4, wobei die mindestens zwei ersten Lagersegmente (33A) im Wesentlichen identisch aufgebaut sind; und/oder
wobei die mindestens zwei zweiten Lagersegmente (33B) im Wesentlichen identisch aufgebaut sind; und/oder
wobei die mindestens zwei ersten Lagersegmente (33A) in einer Umfangsrichtung um die Längsachse im Wesentlichen gleich beabstandet sind; und/oder
wobei die mindestens zwei zweiten Lagersegmente (33B) in einer Umfangsrichtung um die Längsachse im Wesentlichen gleich beabstandet sind.

6. Die ventrikuläre Unterstützungsvorrichtung (1) nach einem der vorstehenden Ansprüche, wobei:
die erste radiale Sensoreinheit eine erste radiale Hall-Sensor-Anordnung umfasst, die konfiguriert ist, um die radiale Position des ersten Endabschnitts (19A) zu bestimmen; und/oder wobei
die zweite radiale Sensoreinheit eine zweite radiale Hall-Sensor-Anordnung umfasst, die konfiguriert ist, um die radiale Position des zweiten Endabschnitts (19B) zu bestimmen.

7. Die ventrikuläre Unterstützungsvorrichtung (1) nach Anspruch 6,
wobei die erste radiale Hall-Sensor-Anordnung Folgendes umfasst: einen ersten Permanentmagneten (193A), der am ersten Endabschnitt (19A) befestigt ist, und mindestens einen ersten radialen Hall-Sensor (38A), der in einer radialen Richtung relativ zur Längsachse benachbart zum ersten Permanentmagneten (193A) angeordnet ist, und/oder
wobei die zweite radiale Hall-Sensor-Anordnung Folgendes umfasst: einen zweiten Permanentmagneten (193B), der am zweiten Endabschnitt (19B) befestigt ist, und mindestens einen zweiten radialen Hall-Sensor (38B), der in der radialen Richtung relativ zur Längsachse benachbart zum zweiten Permanentmagneten (193B) angeordnet ist.

8. Die ventrikuläre Unterstützungsvorrichtung (1) nach einem der vorstehenden Ansprüche, wobei die erste radiale Sensoreinheit konfiguriert ist, um die bestimmte radiale Position des ersten Endabschnitts (19A) an die Steuereinheit zu liefern,
wobei die Steuereinheit konfiguriert ist, um das erste radiale Magnetlager (31A) des ersten aktiven Magnetlagers (30A) zu steuern, um auf der Grundlage der bestimmten radialen Position des ersten Endabschnitts (19A) eine Magnetkraft auf den ersten Endabschnitt (19A) einstellbar zu erzeugen; und/oder
wobei die zweite radiale Sensoreinheit konfiguriert ist, um die bestimmte radiale Position des zweiten Endabschnitts (19B) an die Steuereinheit zu liefern, und
wobei die Steuereinheit konfiguriert ist, um das zweite radiale Magnetlager (31B) des zweiten aktiven Magnetlagers (30B) zu steuern, um auf der Grundlage der ermittelten radialen Position des zweiten Endabschnitts (19B) eine Magnetkraft auf den zweiten Endabschnitt (19B) einstellbar zu erzeugen.

9. Die ventrikuläre Unterstützungsvorrichtung (1) nach einem der vorstehenden Ansprüche, wobei das erste radiale Magnetlager (31A) eines von einem homopolaren Magnetlager und einem heteropolaren Magnetlager ist, und/oder
wobei das zweite radiale Magnetlager (31B) eines von einem homopolaren Magnetlager und einem heteropolaren Magnetlager ist.

10. Die ventrikuläre Unterstützungsvorrichtung (1) nach einem der vorstehenden Ansprüche, wobei die ventrikuläre Unterstützungsvorrichtung (1) eine axiale Sensoranordnung (23) umfasst, die konfiguriert ist, um eine axiale Position der Rotorwelle (10) entlang der Längsachse zu bestimmen, und optional
wobei die axiale Sensoranordnung (23) Folgendes umfasst:
einen ringförmigen Permanentmagneten (15), der an der Rotorwelle (10) in einer Umfangsrichtung um die Rotorwelle (10) herum befestigt ist,
einen axialen Hall-Sensor (23A), der benachbart zum ringförmigen Permanentmagneten (15) in einer Richtung parallel zur Längsachse angeordnet ist, wobei der axiale Hall-Sensor (23A) konfiguriert ist, um die axiale Position der Rotorwelle (10) zu bestimmen.

11. Die ventrikuläre Unterstützungsvorrichtung (1) nach Anspruch 10, wobei das erste aktive Magnetlager (30A) ein erstes axiales Magnetlager (32A) umfasst, das konfiguriert ist, um die axiale Position der Rotorwelle (10) entlang der Längsachse einzustellen, und/oder
wobei das zweite aktive Magnetlager (30B) ein zweites axiales Magnetlager (32B) umfasst, das konfiguriert ist, um die axiale Position der Rotorwelle (10) entlang der Längsachse einzustellen;
und optional:
wobei die bestimmte axiale Position an die Steuereinheit geliefert wird, wobei die Steuereinheit konfiguriert ist, um das erste axiale Magnetlager (32A) des ersten aktiven Magnetlagers (30A) und/oder das zweite axiale Magnetlager (32B) des zweiten aktiven Magnetlagers (30B) zu steuern, um die axiale Position der Rotorwelle (10) auf der Grundlage der bestimmten axialen Position der Rotorwelle (10) einzustellen.

12. Die ventrikuläre Unterstützungsvorrichtung (1) nach einem der vorstehenden Ansprüche, wobei die Steuereinheit Folgendes umfasst:
- einen Sender, der konfiguriert ist, um Daten an eine entfernte Vorrichtung zu senden, wobei die Steuereinheit vorzugsweise konfiguriert ist, um eine Fehlfunktion der ventrikulären Unterstützungsvorrichtung (1) zu erfassen und anschließend auf der Grundlage der erfassten Fehlfunktion einen Alarm zu senden; und/oder
- einen Empfänger, der konfiguriert ist, um Daten von einer entfernten Vorrichtung zu empfangen; und/oder
- ein Datenspeichergerät, wobei das Datenspeichergerät konfiguriert ist, um historische Betriebsdaten der ventrikulären Unterstützungsvorrichtung (1) zu speichern.

13. Die ventrikuläre Unterstützungsvorrichtung (1) nach einem der vorstehenden Ansprüche, wobei eine Geometrie der ventrikulären Unterstützungsvorrichtung (1) konfiguriert ist, um Strömungsfeld eines Fluids, das durch die ventrikuläre Unterstützungsvorrichtung (1) gepumpt wird, keine Totwasserzonen bzw. stagnierenden Strömungszonen (*dead water zones*) umfasst, wobei die Geometrie der ventrikulären Unterstützungsvorrichtung (1) vorzugsweise so konfiguriert ist, dass das von der ventrikulären Unterstützungsvorrichtung (1) gepumpte Fluid nicht über scharfe Kanten der ventrikulären Unterstützungsvorrichtung (1) fließt; und/oder
wobei der Magnetmotor so konfiguriert ist, dass er eine Rotation des Laufrades (11) in mindestens einem von einem pulsatilen Betriebsmodus, einem gegen-pulsatilen Betriebsmodus und einem kontinuierlichen Betriebsmodus bewirkt; und/oder
wobei die ventrikuläre Unterstützungsvorrichtung (1) eine Energieeinheit umfasst, die konfiguriert ist, um die ventrikuläre Unterstützungsvorrichtung (1) mit Energie zu versorgen, wobei die Energieeinheit Folgendes umfasst: mindestens eines von einer Energie-Empfangseinheit, die konfiguriert ist, um Energie vorzugsweise drahtlos und transkutan zu empfangen, und einer Energie-Speichereinheit, die konfiguriert ist, um Energie zu speichern.

14. Die ventrikuläre Unterstützungsvorrichtung (1) nach einem der vorstehenden Ansprüche, wobei der Magnetmotor ein bürstenloser Gleichstrommotor ist, wobei der bürstenlose Gleichstrommotor vorzugsweise einen großen Luftspalt aufweist, und/oder
wobei das erste aktive Magnetlager (30A) und das zweite aktive Magnetlager (30B) im Wesentlichen identisch aufgebaut sind; und/oder
wobei das Blutgefäß eines von einer Vene und einer Arterie eines Benutzers ist, vorzugsweise eine Lungenarterie oder eine Aorta des Benutzers.

15. Die ventrikuläre Unterstützungsvorrichtung (1) nach einem der vorstehenden Ansprüche, wobei die ventrikuläre Unterstützungsvorrichtung (1) an einer äußeren Oberfläche der ventrikulären Unterstützungsvorrichtung (1) ein oder mehrere Anbringungselemente umfasst, die konfiguriert sind, um die ventrikuläre Unterstützungsvorrichtung (1) am Blutgefäß zu fixieren; und/oder
wobei die ventrikuläre Unterstützungsvorrichtung (1) konfiguriert ist, um durch ein oder mehrere Fixierungselemente (40), die außerhalb des Lumens (L) des Blutgefäßes angeordnet sind, am Blutgefäß fixierbar zu sein; und/oder
wobei die ventrikuläre Unterstützungsvorrichtung (1) konfiguriert ist, um vollständig in das Lumen (L) des Blutgefäßes implantiert zu werden.

## Revendications

1. Un dispositif d'assistance ventriculaire (1) destiné à être implanté dans une lumière (L) d'un vaisseau sanguin, comprenant :
une turbine (*impeller*) (11) fixée à un arbre de rotor (10), sachant que la turbine (11) est configurée pour tourner autour d'un axe longitudinal de l'arbre de rotor (10) ;
une unité d'entraînement (20) comprenant un moteur magnétique configuré pour provoquer la rotation de la turbine (11) autour de l'axe longitudinal ;
un premier palier magnétique actif (30A) configuré pour porter une première section d'extrémité (19A) de l'arbre de rotor (10) par rapport à l'unité d'entraînement (20) ;
un deuxième palier magnétique actif (30B) configuré pour supporter une deuxième section d'extrémité (19B) de l'arbre de rotor (10) par rapport à l'unité d'entraînement (20) ; et
une unité de commande configurée pour commander le moteur magnétique, le premier palier magnétique actif (30A) et le deuxième palier magnétique actif (30B),
sachant que le premier palier magnétique actif (30A) comprend :
un premier palier magnétique radial (31A) configuré pour ajuster une position radiale de la première section d'extrémité (19A) par rapport au premier palier magnétique radial (31A),
une première unité de capteur radial configurée pour déterminer la position radiale de la première section d'extrémité (19A) ; et/ou
sachant que le deuxième palier magnétique actif (30B) comprend :
un deuxième palier magnétique radial (31B) configuré pour ajuster une position radiale de la deuxième section d'extrémité (19B) par rapport au deuxième palier magnétique radial (31B),
une deuxième unité de capteur radial configurée pour déterminer la position radiale de la deuxième section d'extrémité (19B).

2. Le dispositif d'assistance ventriculaire (1) d'après la revendication 1, sachant que l'unité de commande est configurée pour :
commander le moteur magnétique pour générer de manière ajustable une force magnétique sur l'arbre de rotor (10) afin de commander une vitesse de rotation de la turbine (11) ; et/ou
commander le premier palier magnétique actif (30A) pour générer de manière ajustable une force magnétique sur la première section d'extrémité (19A) afin de commander une première position de la première section d'extrémité (19A) par rapport au premier palier magnétique actif (30A) ; et/ou
commander le deuxième palier magnétique actif (30B) pour générer de manière ajustable une force magnétique sur la deuxième section d'extrémité (19B) afin de commander une deuxième position de la deuxième section d'extrémité (19B) par rapport au deuxième palier magnétique actif (30B).

3. Le dispositif d'assistance ventriculaire (1) d'après l'une des revendications précédentes,
sachant que le premier palier magnétique radial (31A) comprend au moins deux premiers segments de palier (33A),
sachant que la première unité de capteur radial comprend un premier capteur radial configuré pour mesurer une capacité entre chacun des premiers segments de palier (33A) et la première section d'extrémité (19A),
sachant que le premier capteur radial est configuré pour déterminer la position radiale de la première section d'extrémité (19A) sur la base de la capacité mesurée entre chacun des premiers segments de palier (33A) et la première section d'extrémité (19A) ; et/ou
sachant que le deuxième palier magnétique radial (31B) comprend au moins deux deuxièmes segments de palier (33B),
sachant que la deuxième unité de capteur radial comprend un deuxième capteur radial configuré pour mesurer une capacité entre chacun des segments de deuxième palier (33B) et la deuxième section d'extrémité (19B),
sachant que le deuxième capteur radial est configuré pour déterminer la position radiale de la deuxième section d'extrémité (19B) sur la base de la capacité mesurée entre chacun des deuxièmes segments de palier (33B) et la deuxième section d'extrémité (19B).

4. Le dispositif d'assistance ventriculaire (1) d'après la revendication 3, sachant que chacun des segments de palier premiers et deuxièmes (33A, 33B) comprend :
une culasse magnétique (37A, 37B) agencée de manière adjacente à la première et à la deuxième section d'extrémité (19A, 19B), respectivement ; et
une bobine magnétique radiale (36A, 36B), sachant que la bobine magnétique radiale (36A, 36B) est enroulée autour de la culasse magnétique (37A, 37B).

5. Le dispositif d'assistance ventriculaire (1) d'après la revendication 3 ou 4, sachant que les au moins deux premiers segments de palier (33A) sont construits de façon essentiellement identique ; et/ou
sachant que les au moins deux deuxièmes segments de palier (33B) sont construits de manière essentiellement identique ; et/ou
sachant que les au moins deux premiers segments de palier (33A) sont essentiellement également espacés dans une direction circonférentielle autour de l'axe longitudinal ; et/ou
sachant que les au moins deux deuxièmes segments de palier (33B) sont essentiellement également espacés dans une direction circonférentielle autour de l'axe longitudinal.

6. Le dispositif d'assistance ventriculaire (1) d'après l'une des revendications précédentes, sachant que :
la première unité de capteur radial comprend un premier agencement de capteur à effet Hall radial configuré pour déterminer la position radiale de la première section d'extrémité (19A) ; et/ou
la deuxième unité de capteur radial comprend un deuxième agencement de capteur à effet Hall radial configuré pour déterminer la position radiale de la deuxième section d'extrémité (19B).

7. Le dispositif d'assistance ventriculaire (1) d'après la revendication 6, sachant que le premier agencement de capteur à effet Hall radial comprend un premier aimant permanent (193A) fixé à la première section d'extrémité (19A), et au moins un premier capteur à effet Hall radial (38A) disposé de manière adjacente au premier aimant permanent (193A) dans une direction radiale par rapport à l'axe longitudinal, et/ou
sachant que le deuxième agencement de capteur à effet Hall radial comprend un deuxième aimant permanent (193B) fixé à la deuxième section d'extrémité (19B), et au moins un deuxième capteur à effet Hall radial (38B) disposé de manière adjacente au deuxième aimant permanent (193B) dans la direction radiale par rapport à l'axe longitudinal.

8. Le dispositif d'assistance ventriculaire (1) d'après l'une des revendications précédentes, sachant que la première unité de capteur radial est configurée pour fournir la position radiale déterminée de la première section d'extrémité (19A) à l'unité de commande,
sachant que l'unité de commande est configurée pour commander le premier palier magnétique radial (31A) du premier palier magnétique actif (30A) afin de générer de manière ajustable une force magnétique sur la première section d'extrémité (19A) sur la base de la position radiale déterminée de la première section d'extrémité (19A) ; et/ou
sachant que la deuxième unité de capteur radial est configurée pour fournir la position radiale déterminée de la deuxième section d'extrémité (19B) à l'unité de commande, et
sachant que l'unité de commande est configurée pour commander le deuxième palier magnétique radial (31B) du deuxième palier magnétique actif (30B) afin de générer de manière ajustable une force magnétique sur la deuxième section d'extrémité (19B) sur la base de la position radiale déterminée de la deuxième section d'extrémité (19B).

9. Le dispositif d'assistance ventriculaire (1) d'après l'une des revendications précédentes, sachant que le premier palier magnétique radial (31A) est l'un parmi un palier magnétique homopolaire et un palier magnétique hétéropolaire, et/ou
sachant que le deuxième palier magnétique radial (31B) est l'un parmi un palier magnétique homopolaire et un palier magnétique hétéropolaire.

10. Le dispositif d'assistance ventriculaire (1) d'après l'une des revendications précédentes, sachant que le dispositif d'assistance ventriculaire (1) comprend un agencement de capteur axial (23) configuré pour déterminer une position axiale de l'arbre de rotor (10) le long de l'axe longitudinal, et, facultativement,
sachant que l'agencement de capteur axial (23) comprend :
un aimant permanent en forme d'anneau (15) fixé à l'arbre de rotor (10) dans une direction circonférentielle autour de l'arbre de rotor (10),
un capteur à effet Hall axial (23A) agencé de manière adjacente à l'aimant permanent en forme d'anneau (15) dans une direction parallèle à l'axe longitudinal, sachant que le capteur à effet Hall axial (23A) est configuré pour déterminer la position axiale de l'arbre de rotor (10).

11. Le dispositif d'assistance ventriculaire (1) d'après la revendication 10, sachant que le premier palier magnétique actif (30A) comprend un premier palier magnétique axial (32A) configuré pour ajuster la position axiale de l'arbre de rotor (10) le long de l'axe longitudinal, et/ou
sachant que le deuxième palier magnétique actif (30B) comprend un deuxième palier magnétique axial (32B) configuré pour ajuster la position axiale de l'arbre de rotor (10) le long de l'axe longitudinal ;
et, facultativement :
sachant que la position axiale déterminée est fournie à l'unité de commande, sachant que l'unité de commande est configurée pour commander le premier palier magnétique axial (32A) du premier palier magnétique actif (30A) et/ou le deuxième palier magnétique axial (32B) du deuxième palier magnétique actif (30B) pour ajuster la position axiale de l'arbre de rotor (10) sur la base de la position axiale déterminée de l'arbre de rotor (10).

12. Le dispositif d'assistance ventriculaire (1) d'après l'une des revendications précédentes, sachant que l'unité de commande comprend :
- un émetteur configuré pour transmettre des données à un dispositif distant, sachant que l'unité de commande est de préférence configurée pour détecter un dysfonctionnement du dispositif d'assistance ventriculaire (1) et transmettre ensuite une alerte sur la base du dysfonctionnement détecté ; et/ou
- un récepteur configuré pour recevoir des données de la part d'un dispositif distant ; et/ou
- un dispositif de stockage de données, sachant que le dispositif de stockage de données est configuré pour stocker des données opérationnelles historiques du dispositif d'assistance ventriculaire (1).

13. Le dispositif d'assistance ventriculaire (1) d'après l'une des revendications précédentes, sachant qu'une géométrie du dispositif d'assistance ventriculaire (1) est configurée de manière qu'un champ d'écoulement d'un fluide pompé par le dispositif d'assistance ventriculaire (1) ne comprenne aucune zone de stagnation de flux ou encore zone à faible écoulement (*dead water zones*), sachant que la géométrie du dispositif d'assistance ventriculaire (1) est de préférence configurée de manière que le fluide pompé par le dispositif d'assistance ventriculaire (1) ne s'écoule pas sur des bords tranchants du dispositif d'assistance ventriculaire (1) ; et/ou
sachant que le moteur magnétique est configuré pour provoquer la rotation de la turbine (11) dans au moins un mode de fonctionnement pulsatile, un mode de fonctionnement contre-pulsatile et un mode de fonctionnement continu ; et/ou
sachant que le dispositif d'assistance ventriculaire (1) comprend une unité d'alimentation configurée pour fournir de l'énergie au dispositif d'assistance ventriculaire (1), sachant que l'unité d'alimentation comprend au moins l'une parmi une unité de réception d'énergie configurée, de préférence sans fil et de manière transcutanée, pour recevoir de l'énergie, et une unité de stockage d'énergie configurée pour stocker de l'énergie.

14. Le dispositif d'assistance ventriculaire (1) d'après l'une des revendications précédentes, sachant que le moteur magnétique est un moteur à courant continu sans balais, sachant que le moteur à courant continu sans balais présente de préférence un grand entrefer, et/ou
sachant que le premier palier magnétique actif (30A) et le deuxième palier magnétique actif (30B) sont de construction essentiellement identique ; et/ou
sachant que le vaisseau sanguin est l'une parmi une veine et une artère d'un utilisateur, de préférence une artère pulmonaire ou une aorte de l'utilisateur.

15. Le dispositif d'assistance ventriculaire (1) d'après l'une des revendications précédentes, sachant que le dispositif d'assistance ventriculaire (1) comprend un ou plusieurs éléments d'attache (*attachment elements*) sur une surface extérieure du dispositif d'assistance ventriculaire (1) configurés pour fixer le dispositif d'assistance ventriculaire (1) au vaisseau sanguin ; et/ou
sachant que le dispositif d'assistance ventriculaire (1) est configuré pour pouvoir être fixé au vaisseau sanguin par un ou plusieurs éléments de fixation (40) agencés à l'extérieur de la lumière (L) du vaisseau sanguin ; et/ou
sachant que le dispositif d'assistance ventriculaire (1) est configuré pour être totalement implanté dans la lumière (L) du vaisseau sanguin.
